# EUROPEAN PATENT APPLICATION

(11) **EP 4 516 775 A1**
(43) Date of publication of application: **05.03.2025**
(21) Application number: 22939700.5
(22) Date of filing: 23.08.2022
(51) Int. Cl.: C07C 269/04, C07C 271/16, C07C 213/00, C07D 263/22, C07C 269/06, C07D 203/20, C07D 203/02, C07C 271/14, C07C 215/28

(54) **SACUBITRIL INTERMEDIATE, PREPARATION METHOD THEREFOR, AND USE THEREOF**

(30) Priority: 29.04.2022 CN 202210466737
(71) Applicant: Shenzhen Catalyst Technology Co., Ltd, Shenzhen, Guangdong 518129 (CN); Shenzhen Greencat Pharmaceutical Technology Co., Ltd., Shenzhen, Guangdong 518129 (CN)
(72) Inventor: LANG, Qiwei, Shenzhen, Guangdong 518129 (CN); ZHAO, Jinhui, Shenzhen, Guangdong 518129 (CN); LIU, Chuangji, Shenzhen, Guangdong 518129 (CN); ZHANG, Ziheng, Shenzhen, Guangdong 518129 (CN); DING, Xiaobing, Shenzhen, Guangdong 518129 (CN)
(74) Representative: Zaboliene, Reda
(86) International application number: PCT/CN2022/114100
(87) International publication number: WO 2023/206874

(57) **Abstract**

A sacubitril intermediate, a preparation method therefor, and use thereof. A key intermediate N-Boc amino alcohol represented by formula (10) or formula (10-a) can be efficiently prepared. The intermediate can be used to prepare a neutral endopeptidase (NEP) inhibitor or a prodrug thereof, particularly a NEP inhibitor comprising a skeleton of γ-amino-δ-biphenyl-α-methylalkanoic acid or ester, such as sacubitril. Also provided are intermediates for preparing formula (10) or formula (10-a). Raw materials of the process route are cheap, the operation is simple and convenient, the production cost is low, and the method is suitable for industrial production

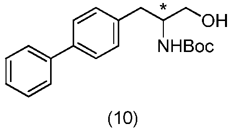

## Description

### Technical field

The present disclosure falls within the field of pharmaceuticals, relates to a pharmaceutical intermediate, and particularly relates to a sacubitril intermediate, a preparation method therefor, and use thereof.

### Background

Mammalian endogenous atrial natriuretic peptide (ANP), also known as atrial natriuretic factor (ANF), has diuretic, natriuretic, and vasodilatory functions. Natural ANF peptides are metabolically inactivated, in particular by degradative enzymes considered equivalent to NEP, which also cause metabolic inactivation of enkephalins.

Sacubitril (AHU-377) is one of the major components of LCZ696 (CAS: 936623-90-4), a medicament developed by Novartis for a heart failure treatment. This medicament for the heart failure treatment is a supramolecular complex (compound) formed by combining valsartan and sacubitril (AHU-377) through a non-covalent bond. The medicament has dual functions of angiotensin receptor blocking and neutral endopeptidase inhibition, reduces the risk of cardiovascular diseases, is mainly used for treating heart failure, and can also be used for hypertension.

Sacubitril (AHU-377) is typically prepared via a key intermediate N-Boc aminoalcohol [formula (10-a)] having a chemical name: *tert*-butyl N-[(1*R*)-2-[1,1'-biphenyl]-4-yl-1-(hydroxymethyl)ethyl]carbamate (CAS: 1426129-50-1); and a structural formula thereof is as follows:

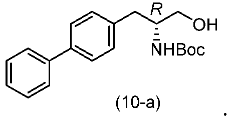

In the prior art, there are many patent documents on the synthesis methods for N-Boc aminoalcohol, a sacubitril intermediate, such as the literature J. Med. Chem. 1995, 38, 1689-1700, which reports a method for preparing a sacubitril intermediate using D-tyrosine as a starting material. The synthetic route is as follows:

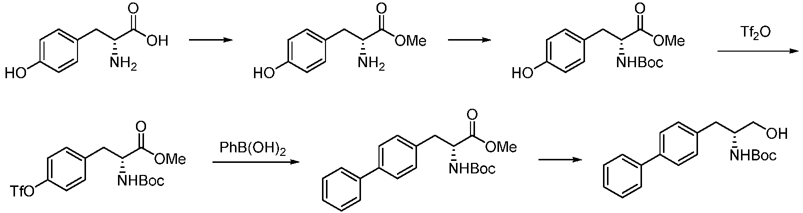

The D-tyrosine used by the method is non-natural amino acid and is expensive; expensive trifluoromethanesulfonic anhydride reagent is also used in the reaction process, and the reagent is active and highly corrosive, has high requirements on production equipment and operation, and is not conducive to the industrial application.

Patents WO2014032627 and CN 105026361 disclose a preparation method for N-Boc aminoalcohol, and the synthetic route is as follows:

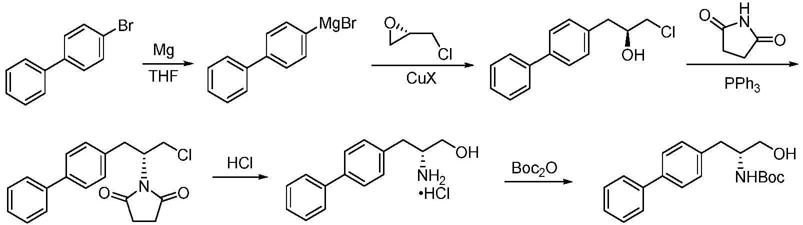

The method mainly faces problems such as: difficulties in separation and purification, and an increase in solid waste caused by the generation of a large amount of triphenylphosphine oxide compounds after the reaction using triphenylphosphine; the method also involves the use of azodicarboxylate compounds, which are sensitive to light, heat, and vibration, presenting potential dangers during the heating process, and these issues will significantly raise the overall production costs.

Similarly, patent CN 105985225 discloses a preparation method for a sacubitril intermediate, and the specific information is as follows:

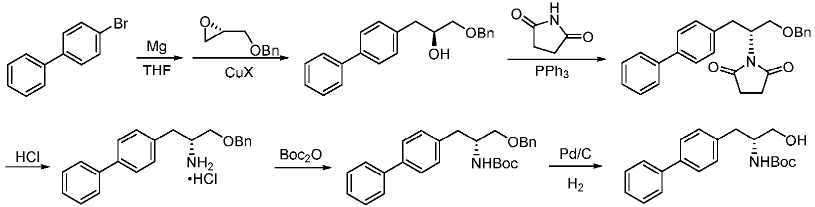

The method is similar to that disclosed in patents WO2014/032627 and CN 105026361, with the main difference being the use of a hydroxyl protective reagent instead of epichlorohydrin. The reaction processes are substantially similar, substantially consistent reagents are used for the same reaction types, and in the final step, because the hydroxyl is protected by benzyl, additional palladium-catalyzed hydrogenolysis is required for removing the protective group. Although this patent reports an increase in the yield of N-Boc aminoalcohol preparation, the final addition of a noble metal-catalyzed hydrogenolysis to remove the benzyl group, considering the cost of the reagents, can lead to a significant cost increase.

Patents WO 2013/026773 and CN 103764624 disclose a method for preparing a sacubitril intermediate aminoalcohol using *p*-phenyl benzaldehyde as a starting material, and the synthetic route is as follows:

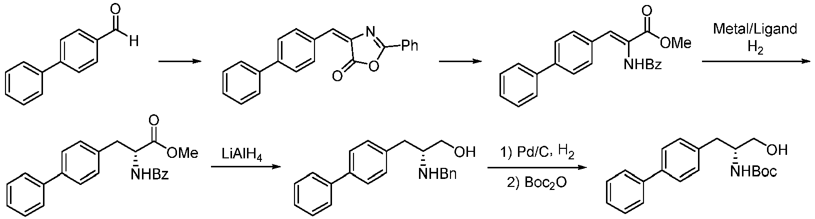

The process includes a catalytic hydrogenation step, which has the disadvantage of using noble metals Rh and Pd in large amounts, resulting in high production costs.

In addition to the chemical synthesis methods described above for preparing the N-Boc aminoalcohol intermediate, the intermediate may also be prepared by enzymatic methods. For example, patent CN 105884656 discloses a method for preparing the intermediate by enzyme-catalyzed asymmetric reductive amination, and the specific information is as follows:

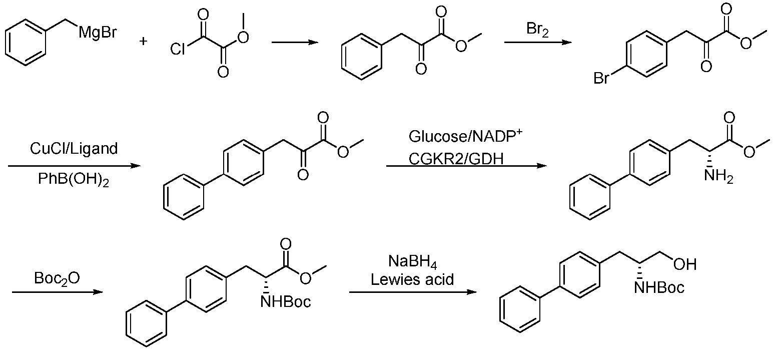

In the method, benzylmagnesiumbromide is used as a starting material and reacts with methyl oxalyl chloride to generate keto ester; then, under an action of a brominating reagent, bromination is performed on the 4-position of a benzene ring; copper catalysis is used for coupling with phenylboronic acid to give biphenyl keto ester; the keto ester is then subjected to catalytic asymmetric reductive amination in a system of glucose, NADP⁺, and reductase CGKR2 and GDH to give chiral amino acid methyl ester; and after the amino is protected by Boc, methyl carboxylate is reduced to an alcohol under an action of sodium borohydride and Lewis acid to give the key intermediate N-Boc aminoalcohol.

The method has the problems of relatively long synthetic route, inconvenient use of acyl chloride and brominating reagents, and relatively large amounts of metal copper and reductase needed in the steps of copper-catalyzed coupling and asymmetric reductive amination; furthermore, there is no indication in the patent of enantiomeric excess of the product after reductive amination.

In summary, in the preparation processes reported in the prior art, in one aspect, the preparation of the key chiral intermediate N-Boc aminoalcohol (10-a) of sacubitril is limited by starting materials, reaction reagents, post-treatment processes, and the like. In another aspect, the problems of the long synthetic route, low diastereoisomer ratio, environmental unfriendliness, and the like result in high production cost, complex operation, and unfavorable industrialization. Therefore, the development of a more simple and convenient, cost-effective, and industrially viable production process for the key chiral intermediate *N*-Boc aminoalcohol (10-a) is of significant importance.

### Summary

The present disclosure relates to a preparation process for a sacubitril intermediate, and by implementing the method disclosed in the present disclosure, the key intermediate N-Boc aminoalcohol [formula (10) and formula (10-a)] can be efficiently prepared. The intermediate can be used for a neutral endopeptidase (NEP) inhibitor or a prodrug thereof, particularly a NEP inhibitor containing a backbone of γ-amino-δ-biphenyl-α-methylalkanoic acid or ester, such as sacubitril and the like. This process route has the advantages of cheap starting materials, simple and convenient operation, no special requirements on equipment, and low production costs, is suitable for industrial production, and has great application potential and commercial value. Moreover, the present disclosure also provides an intermediate for preparing compound (10-a).

Specifically, in one aspect, the present disclosure provides a preparation process for compound (10), which comprises the following steps: 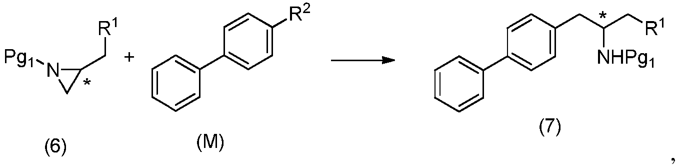
step a: reacting compound (6) with compound (M) under an action of a catalyst to give compound (7), wherein
"*" represents that compound (6) and compound (7) are both in R configuration or S configuration; preferably, "*" represents that compound (6) and compound (7) are both in R configuration;
R¹ is F, Cl, Br, I, or -OR³, and R³ represents C₁₋₆ alkyl or C₁₋₆ heteroalkyl;
R² is 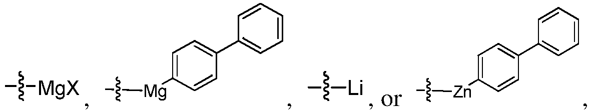 wherein X is Cl, Br, or I;
Pg₁ is an amino protective group; and
compound (10) has a structure: 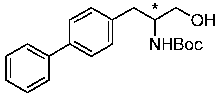 wherein "*" represents that compound (10) is in R configuration or S configuration, preferably, "*" represents that compound (10) is in R configuration.

In some embodiments, in step a of the process of the present disclosure,
the catalyst is a cuprous salt, preferably CuI, CuBr, CuCl, or CuCN; the amino protective group is benzyloxycarbonyl, *tert*-butoxycarbonyl, methoxycarbonyl, ethoxycarbonyl, isopropyloxycarbonyl, isobutyloxycarbonyl, fluorenylmethoxycarbonyl, allyloxycarbonyl, or trimethylsilylethoxycarbonyl; or
the reaction is performed under dry and oxygen-free conditions; preferably, the reaction is performed under dry and oxygen-free conditions with introduction of nitrogen for protection; or
the reaction temperature of the reaction is -60 to 0 °C; preferably, the reaction temperature is -45 to 0 °C; more preferably, the reaction temperature is -30 to -10 °C; most preferably, the reaction temperature is -20 to -15 °C; or
the reaction time of the reaction is 0.5-3 h; preferably, the reaction time is 0.8-2 h; more preferably, the reaction time is 1-1.5 h; most preferably, the reaction time is 1 h.

In some embodiments, in step a of the process of the present disclosure, the reaction solvent of the reaction is a first solvent, and the first solvent is an organic aprotic solvent; preferably, the organic aprotic solvent is tetrahydrofuran, 2-methyltetrahydrofuran, toluene, cyclopentyl methyl ether, dichloromethane, methyl *tert*-butyl ether, or diethyl ether, or any combination thereof.

In some embodiments, in step a of the process of the present disclosure, the feeding molar ratio of compound (6) to compound (M) is 1:0.7-2, preferably 1:1-2, more preferably 1:1-1.5, and most preferably 1:1.

In some embodiments, the process of the present disclosure further comprises the following steps: 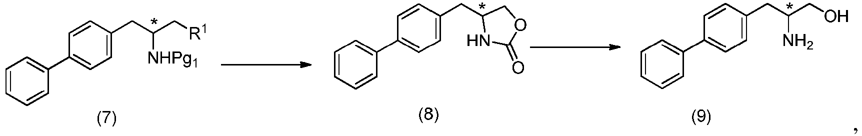
wherein "*" represents that compound (7), compound (8), and compound (9) are all in R configuration or S configuration; preferably, "*" represents that compound (7), compound (8), and compound (9) are all in R configuration;
step b: reacting compound (7) in a second solvent under a first heating condition to give compound (8), wherein
preferably, the reaction time is 0.3-2 h; more preferably, the reaction time is 0.3-1 h; most preferably, the reaction time is 0.5 h;
step c: subjecting compound (8) and a base to a hydrolysis reaction in a third solvent under a second heating condition to give compound (9), wherein
preferably, the base is sodium hydroxide, potassium hydroxide, lithium hydroxide, potassium *tert*-butoxide, sodium *tert*-butoxide, sodium carbonate, potassium carbonate, or sodium methoxide, or any combination thereof;
preferably, the feeding molar ratio of compound (8) to the base is 1:(0.9-5); more preferably, the feeding molar ratio of compound (8) to the base is 1:(1-4); even more preferably, the feeding molar ratio of compound (8) to the base is 1:(2-4); most preferably, the feeding molar ratio of compound (8) to the base is 1:3.75; and
preferably, the reaction time is 1-5 h; more preferably, the reaction time is 2-4 h; most preferably, the reaction time is 3 h.

In some embodiments, in step b and step c of the process of the present disclosure, the heating temperatures under the first heating condition and the second heating condition are each independently 80-150 °C, preferably 100-150 °C, further preferably 110-130 °C, and most preferably 120 °C; preferably, the heating temperatures under the first heating condition and the second heating condition are the same temperature; or
the second solvent and the third solvent are each independently *n*-butanol, benzene, toluene, ethylene glycol dimethyl ether, *N*,*N*-dimethylformamide, and *N*,*N*-dimethylacetamide; preferably, the second solvent and the third solvent are the same solvent.

In some embodiments, in the process of the present disclosure, the reaction of step c is performed by adding a base directly without any post-treatment after the reaction of step b.

In some embodiments, the process of the present disclosure further comprises the following steps: 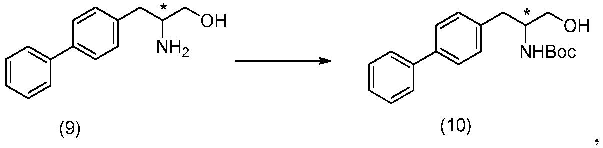
wherein "*" represents that compound (9) and compound (10) are both in R configuration or S configuration; preferably, "*" represents that compound (9) and compound (10) are both in R configuration; and
step d: subjecting compound (9) to an amino protection reaction to give compound (10).

In some embodiments, in step a of the process of the present disclosure, compound (6) is prepared by the following steps: 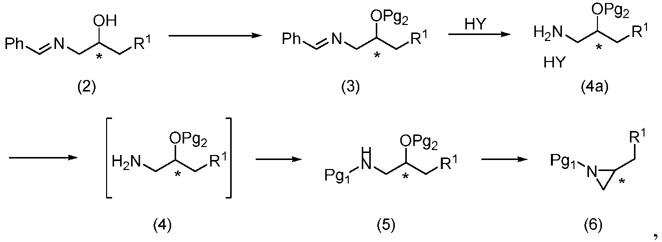
wherein "*" on compound (2), compound (3), compound (4a), compound (4), and compound (5) represents that compound (2), compound (3), compound (4a), compound (4), and compound (5) are all in S configuration, and "*" on compound (6) represents that compound (6) is in R configuration; or "*" on compound (2), compound (3), compound (4a), compound (4), and compound (5) represents that compound (2), compound (3), compound (4a), compound (4), and compound (5) are all in R configuration, and "*" on compound (6) represents that compound (6) is in S configuration;
Pg₂ is a hydroxyl protective group, preferably methanesulfonyl, trifluoromethanesulfonyl, *p*-toluenesulfonyl, or nitrosulfonyl; R¹ and Pg₁ have the definitions according to the present disclosure;
step a': subjecting compound (2) and a hydroxyl protective reagent to a hydroxyl protection reaction to give compound (3); preferably, the feeding molar ratio of compound (2) to the hydroxyl protective reagent is 1:(1-3); more preferably, the feeding molar ratio of compound (2) to the hydroxyl protective reagent is 1:(1-2); even more preferably, the feeding molar ratio of compound (2) to the hydroxyl protective reagent is 1:(1.2-1.5); most preferably, the feeding molar ratio of compound (2) to the hydroxyl protective reagent is 1:1.3;
step b': reacting compound (3) with acid HY to give compound (4a); preferably, the acid HY is hydrochloric acid, hydrobromic acid, formic acid, hydroiodic acid, *p*-toluenesulfonic acid, or trifluoromethanesulfonic acid;
step c': reacting compound (4a) under an action of a base to give compound (4); preferably, the base is sodium hydroxide, potassium hydroxide, sodium carbonate, sodium bicarbonate, potassium bicarbonate, or potassium carbonate;
step d': subjecting compound (4) and an amino protective reagent to an amino protection reaction to give compound (5); preferably, the feeding molar ratio of compound (4) to the amino protective reagent is 1:1-1.5; and
step e': reacting compound (5) in a fourth solvent under an action of a basic reagent to give compound (6); preferably, the basic reagent is sodium hydride, sodium methoxide, sodium hydroxide, potassium hydroxide, potassium *tert*-butoxide, sodium *tert*-butoxide, potassium carbonate, sodium carbonate, or sodium ethoxide; preferably, the feeding molar ratio of compound (5) to the basic reagent is 1:(1-3), more preferably 1:(1.5-2.5), and most preferably 1:2.2; preferably, the fourth solvent is tetrahydrofuran or *N*,*N*-dimethylformamide, or a combination thereof; preferably, the reaction temperature is -10 to 50 °C; further preferably, the reaction temperature is -10 to 15 °C; even more preferably, the reaction temperature is -5 to 10 °C; most preferably, the reaction temperature is 0-10 °C.

In some embodiments, in step a of the process of the present disclosure, compound (6) is prepared by the following steps: 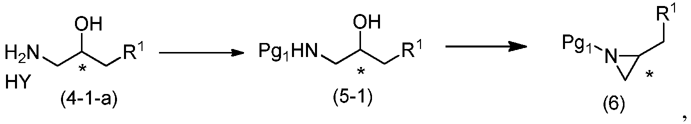
wherein "*" on compound (4-1-a) and compound (5-1) represents that compound (4-1-a) and compound (5-1) are both in S configuration, and "*" on compound (6) represents that compound (6) is in R configuration; or "*" on compound (4-1-a) and compound (5-1) represents that compound (4-1-a) and compound (5-1) are both in R configuration, and "*" on compound (6) represents that compound (6) is in S configuration;
step a": subjecting compound (4-1-a) and an amino protective reagent to an amino protection reaction under an action of a base to give compound (5-1); preferably, the feeding molar ratio of compound (4-1-a) to the amino protective reagent is 1:(0.7-2); preferably, the base is sodium hydroxide, potassium hydroxide, sodium carbonate, or potassium carbonate; and
step b": reacting compound (5-1) at a low temperature under an action of triphenylphosphine and ethyl azodicarboxylate and under nitrogen atmosphere to give compound (6), wherein the low temperature is -40 to 0 °C, preferably -30 to -10 °C, and more preferably -20 to -10 °C.

In some embodiments, in step a of the process of the present disclosure, compound (6) is prepared by the following steps: 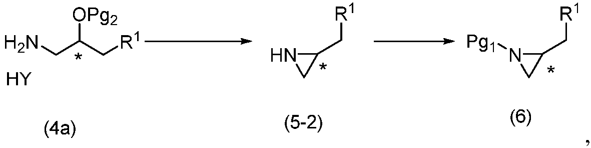 wherein
"*" on compound (4a) represents that compound (4a) is in S configuration, and "*" on compound (5-2) and compound (6) represents that compound (5-2) and compound (6) are both in R configuration; or "*" on compound (4a) represents that compound (4a) is in R configuration, and "*" on compound (5-2) and compound (6) represents that compound (5-2) and compound (6) are both in S configuration;
step a‴: reacting compound (4a) under an action of a base to give compound (5-2); preferably, the base is sodium hydroxide, potassium hydroxide, sodium carbonate, or potassium carbonate; preferably, the feeding molar ratio of compound (4a) to the base is 1:(1-3), more preferably 1:(1.2-2.5), and most preferably 1:1.5; preferably, the reaction temperature is 10-100 °C; more preferably, the reaction temperature is 30-80 °C; even more preferably, the reaction temperature is 40-60 °C; most preferably, the reaction temperature is 50 °C; and
step b"': continuously subjecting compound (5-2) and an amino protective reagent to an amino protection reaction to give compound (6); preferably, the feeding molar ratio of compound (5-2) to the amino protective reagent is 1:(0.7-2); preferably, the reaction is a low-temperature reaction, and the low temperature is -5 to 15 °C; more preferably, the low temperature is 0-10 °C.

In another aspect, the present disclosure provides a compound having a structure of formula (7a) or (7b) below: 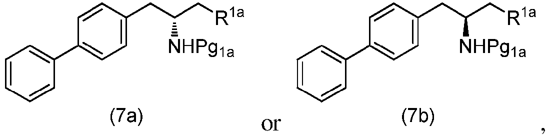
wherein R^{1a} is F, Cl, Br, I, or -OR³, and R³ represents C₁₋₆ alkyl or C₁₋₆ alkyl containing a heteroatom substitution;
Pg₁ₐ is an amino protective group, preferably benzyloxycarbonyl, *tert*-butoxycarbonyl, methoxycarbonyl, ethoxycarbonyl, isopropyloxycarbonyl, isobutyloxycarbonyl, fluorenylmethoxycarbonyl, allyloxycarbonyl, or trimethylsilylethoxycarbonyl;
and when R^{1a} is Br or I, Pg₁ₐ is not *tert*-butoxycarbonyl.

In some embodiments, compound (7a) or compound (7b) of the present disclosure has one of the following structures:

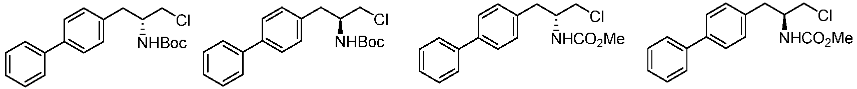

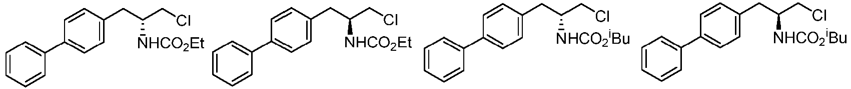

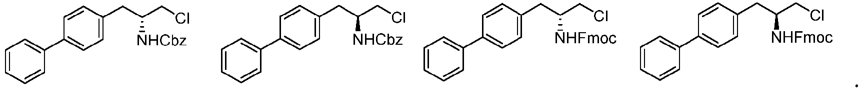

In another aspect, the present disclosure provides a compound having a structure of formula (6a) or (6b) below: 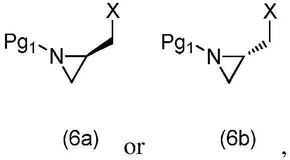
wherein X is F, Cl, Br, or I; and
Pg₁ is an amino protective group, preferably benzyloxycarbonyl, *tert*-butoxycarbonyl, methoxycarbonyl, ethoxycarbonyl, isopropyloxycarbonyl, isobutyloxycarbonyl, fluorenylmethoxycarbonyl, allyloxycarbonyl, or trimethylsilylethoxycarbonyl.

In some embodiments, compound (6a) or compound (6b) of the present disclosure has one of the following structures:

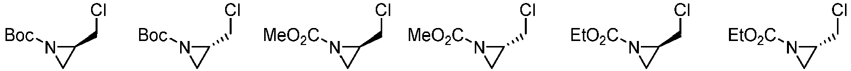

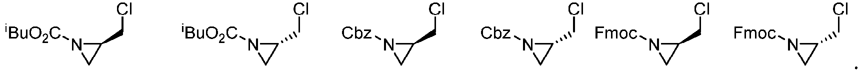

In another aspect, the present disclosure provides a compound having a structure of formula (8-a) or (8-b) below:

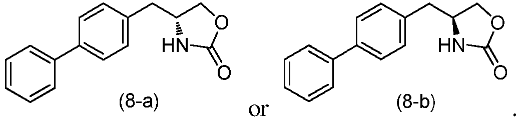

In another aspect, the present disclosure provides use of the process according to the present disclosure or the compound according to the present disclosure in the preparation of sacubitril.

### Detailed specification of the present disclosure

### Definitions of Terms

Certain embodiments of the present disclosure will now be described in detail, and examples of the embodiments are illustrated by the accompanying structural and chemical formulas. The present disclosure is intended to cover all alternatives, modifications, and equivalents, which are included within the scope of the present disclosure as defined by the claims. Those skilled in the art should recognize that many methods and materials similar or equivalent to those described herein can be used in the practice of the present disclosure. The present disclosure is in no way limited to the methods and materials described herein. In the event that one or more of the incorporated literature, patents, and similar materials differ from or contradict the present application (including but not limited to defined terms, term usage, described techniques, and the like), the present application prevails.

Unless otherwise stated, all technical and scientific terms used herein have the same meaning as commonly understood by those skilled in the art to which the present disclosure belongs. All patents and publications referred to herein are incorporated herein by reference in their entirety.

The descriptions "each... be independently" and "... be each independently" and "... be independently" in the present disclosure are used interchangeably and should be understood in a broad sense, and the descriptions may mean that specific items expressed by the same symbol in different groups do not affect each other, or that specific items expressed by the same symbol in the same group do not affect each other.

"C_{q1-q2}" in the present disclosure represents the number of carbon atoms in the group described. For example, C₁₋₆ alkyl represents alkyl having 1-6 carbon atoms.

The term "alkyl" represents a saturated linear or branched monovalent hydrocarbyl group containing 1-20 carbon atoms. In another embodiment, the alkyl group contains 1-6 carbon atoms; in yet another embodiment, the alkyl group contains 1-4 carbon atoms; in still another embodiment, the alkyl group contains 1-3 carbon atoms. Examples of alkyl groups include, but are not limited to, methyl, ethyl, *n*-propyl, isopropyl, *n*-butyl, isobutyl, *sec*-butyl (*s*-Bu, -CH(CH₃)CH₂CH₃), *tert*-butyl, *n*-pentyl, 2-pentyl, and the like.

The term "heteroalkyl" represents that one carbon atom in the alkyl group is substituted with a heteroatom, wherein the heteroatom is O, S, N, or Si. In one embodiment, the heteroalkyl group contains 1-6 carbon atoms; in yet another embodiment, the heteroalkyl group contains 1-4 carbon atoms; in still another embodiment, the heteroalkyl group contains 1-3 carbon atoms. Examples of heteroalkyl groups include, but are not limited to, -CH₂OCH₃, -CH₂OCH₂CH₃, - CH₂SCH₃, -CH₂SiH₂CH₃, -CH₂NHCH₃, and the like.

The term "halogen" represents F (fluorine), Cl (chlorine), Br (bromine), or I (iodine).

The term "amino protective group" refers to a substituent attached to an amino group to block or protect functionality of the amino group in a compound, and examples of amino protective groups include, but are not limited to, benzyloxycarbonyl (-Cbz), *tert-*butoxycarbonyl (-Boc), methoxycarbonyl (-COOMe or -CO₂Me), ethoxycarbonyl (-COOEt or -CO₂Et), isobutyloxycarbonyl (-COO*ⁱ*Bu or -CO₂*ⁱ*Bu), isopropyloxycarbonyl (-COO*ⁱ*Pr or - CO₂*ⁱ*Pr), fluorenylmethoxycarbonyl (Fmoc), allyloxycarbonyl (Alloc), and trimethylsilylethoxycarbonyl (Teoc).

The term "hydroxyl protective group" refers to a substituent of hydroxyl used to block or protect functionality of the hydroxyl, and examples of hydroxyl protective groups include, but are not limited to, methanesulfonyl -(Ms), trifluoromethanesulfonyl (-Tf), *p*-toluenesulfonyl (-Ts), or nitrosulfonyl (-Ns).

The term "first" or "second" or "third" does not indicate an order of appearance, but merely serves to distinguish the modifiers that follow the first or second or third, such as a first solvent, a second solvent, or a third solvent representing the solvent separately used in the corresponding reaction; similarly, a first heating condition and a second heating condition also separately represent heating conditions in the corresponding reactions.

### Detailed description of the present disclosure

By implementing the method disclosed in the present disclosure, the key intermediate N-Boc aminoalcohol [formula (10) or formula (10-a)] can be efficiently prepared. The intermediate can be used to prepare a neutral endopeptidase (NEP) inhibitor or a prodrug thereof, particularly a NEP inhibitor comprising a skeleton of γ-amino-δ-biphenyl-α-methylalkanoic acid or ester, such as sacubitril. Also provided are intermediates for preparing formula (10) or formula (10-a).

Specifically, in one aspect, the present disclosure provides a preparation process for compound (10), which comprises the following steps: 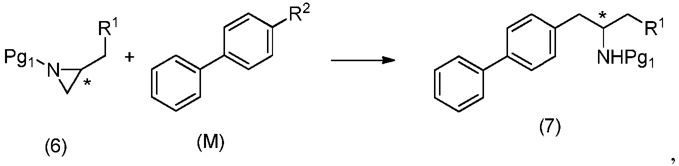
wherein R¹, R², Pg₁, and compound (10) each have the definitions according to the present disclosure;
step a: reacting compound (6) with compound (M) under an action of a catalyst to give compound (7), wherein
"*" represents that compound (6) and compound (7) are both in R configuration or S configuration.

In some preferred embodiments, in step a, "*" represents that compound (6) and compound (7) are both in R configuration, and step a is represented as: 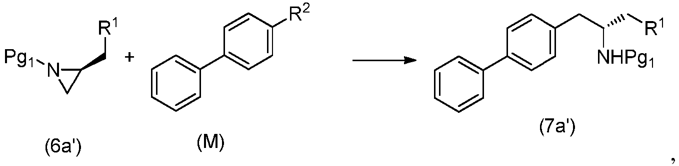 step [a]: reacting compound (6a') with compound (M) under an action of a catalyst to give compound (7a'), wherein R¹, R², and Pg₁ each have the definition according to the present disclosure.

In some embodiments, R¹ is F, Cl, Br, I, or -OR³, and R³ represents C₁₋₆ alkyl or C₁₋₆ heteroalkyl.

In some embodiments, R² is 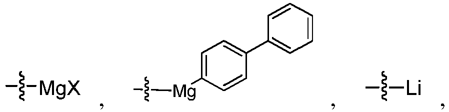 or 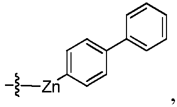 wherein X is Cl, Br, or I.

In some embodiments, Pg₁ is an amino protective group.

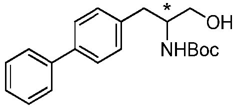

In some embodiments, compound (10) has a structure: (10) , wherein "*" represents that compound (10) is in R configuration or S configuration.

In some preferred embodiments, compound (10) has a structure: 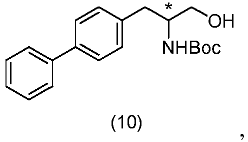 wherein "*" represents that compound (10) is in R configuration, that is, compound (10) is compound (10-a):

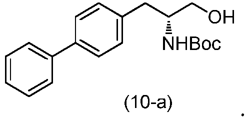

In some embodiments, in step a of the process of the present disclosure, the catalyst is a cuprous salt, preferably CuI, CuBr, CuCl, or CuCN.

In some embodiments, in step a of the process of the present disclosure, the amino protective group is benzyloxycarbonyl, *tert*-butoxycarbonyl, methoxycarbonyl, ethoxycarbonyl, fluorenylmethoxycarbonyl, allyloxycarbonyl, or trimethylsilylethoxycarbonyl.

In some embodiments, in step a of the process of the present disclosure, the reaction is performed under dry and oxygen-free conditions; in some preferred embodiments, in step a of the process of the present disclosure, the reaction is performed under dry and oxygen-free conditions with introduction of nitrogen for protection.

In some embodiments, in step a of the process of the present disclosure, the reaction temperature of the reaction is -60 to 0 °C; in some preferred embodiments, in step a of the process of the present disclosure, the reaction temperature is -45 to 0 °C; in some more preferred embodiments, in step a of the process of the present disclosure, the reaction temperature is -30 to -10 °C; in some most preferred embodiments, in step a of the process of the present disclosure, the reaction temperature is -20 to -15 °C.

In some embodiments, in step a of the process of the present disclosure, the reaction time of the reaction is 0.5-3 h; in some preferred embodiments, in step a of the process of the present disclosure, the reaction time is 0.8-2 h; in some more preferred embodiments, in step a of the process of the present disclosure, the reaction time is 1-1.5 h; in some most preferred embodiments, in step a of the process of the present disclosure, the reaction time is 1 h.

In some embodiments, in step a of the process of the present disclosure, the reaction solvent of the reaction is a first solvent, and the first solvent is an organic aprotic solvent; preferably, the organic aprotic solvent is tetrahydrofuran, 2-methyltetrahydrofuran, toluene, cyclopentyl methyl ether, dichloromethane, methyl *tert*-butyl ether, or diethyl ether, or any combination thereof.

In some embodiments, in step a of the process of the present disclosure, the feeding molar ratio of compound (6) to compound (M) is 1:0.7-2; in some preferred embodiments, in step a of the process of the present disclosure, the feeding molar ratio of compound (6) to compound (M) is 1:1-2; in some more preferred embodiments, in step a of the process of the present disclosure, the feeding molar ratio of compound (6) to compound (M) is 1:1-1.5; in some most preferred embodiments, in step a of the process of the present disclosure, the feeding molar ratio of compound (6) to compound (M) is 1:1.

In some embodiments, the process of the present disclosure further comprises the following steps: 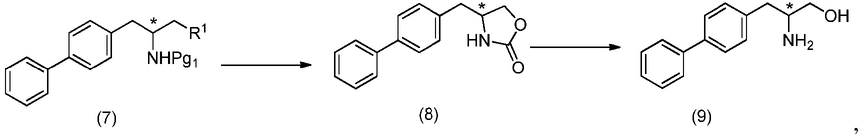
wherein R¹, R², Pg₁, and compound (10) each have the definitions according to the present disclosure;
"*" represents that compound (7), compound (8), and compound (9) are all in R configuration or S configuration; preferably, "*" represents that compound (7), compound (8), and compound (9) are all in R configuration;
step b: reacting compound (7) in a second solvent under a first heating condition to give compound (8),
wherein preferably, the reaction time is 0.3-2 h; more preferably, the reaction time is 0.3-1 h; most preferably, the reaction time is 0.5 h;
step c: subjecting compound (8) and a base to a hydrolysis reaction in a third solvent under a second heating condition to give compound (9), wherein
preferably, the base is sodium hydroxide, potassium hydroxide, lithium hydroxide, potassium *tert*-butoxide, sodium *tert*-butoxide, sodium carbonate, potassium carbonate, or sodium methoxide, or any combination thereof;
preferably, the feeding molar ratio of compound (8) to the base is 1:(0.9-5); more preferably, the feeding molar ratio of compound (8) to the base is 1:(1-4); even more preferably, the feeding molar ratio of compound (8) to the base is 1:(2-4); most preferably, the feeding molar ratio of compound (8) to the base is 1:3.75; and
preferably, the reaction time is 1-5 h; more preferably, the reaction time is 2-4 h; most preferably, the reaction time is 3 h.

In some embodiments, heating temperatures under the first heating condition and the second heating condition are each independently 80-150 °C, preferably 100-150 °C, further preferably 110-130 °C, and most preferably, in some embodiments, in step b and step c of the process of the present disclosure, 120 °C; preferably, heating temperatures under the first heating condition and the second heating condition are the same temperature.

In some embodiments, in step b and step c of the process of the present disclosure, the second solvent and the third solvent are each independently *n*-butanol, benzene, toluene, ethylene glycol dimethyl ether, *N*,*N*-dimethylformamide, or *N*,*N*-dimethylacetamide, or any combination thereof; preferably, the second solvent and the third solvent are the same solvent.

In some embodiments, in the process of the present disclosure, the reaction of step c is performed by adding a base directly without any post-treatment after the reaction of step b.

In some embodiments, the process of the present disclosure further comprises the following steps: 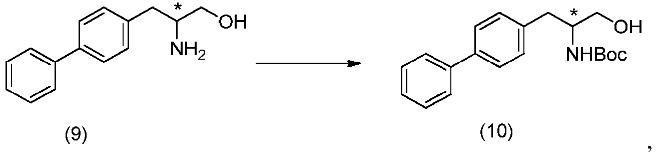
wherein "*" represents that compound (9) and compound (10) are both in R configuration or S configuration; preferably, "*" represents that compound (9) and compound (10) are both in R configuration; and
step d: subjecting compound (9) to an amino protection reaction to give compound (10).

In some embodiments, in step a of the process of the present disclosure, compound (6) is prepared by the following steps: 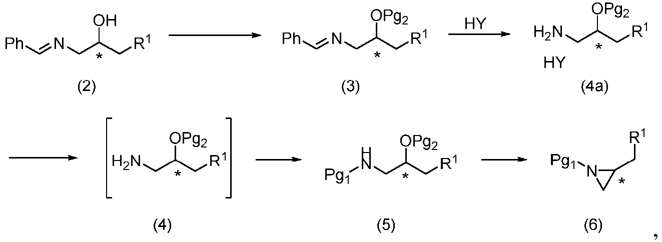
wherein "*" on compound (2), compound (3), compound (4a), compound (4), and compound (5) represents that compound (2), compound (3), compound (4a), compound (4), and compound (5) are all in S configuration, and "*" on compound (6) represents that compound (6) is in R configuration; or "*" on compound (2), compound (3), compound (4a), compound (4), and compound (5) represents that compound (2), compound (3), compound (4a), compound (4), and compound (5) are all in R configuration, and "*" on compound (6) represents that compound (6) is in S configuration;
Pg₂ is a hydroxyl protective group, preferably methanesulfonyl, trifluoromethanesulfonyl, *p*-toluenesulfonyl, or nitrosulfonyl; R¹ and Pg₁ have the definitions according to claim 1;
step a': subjecting compound (2) and a hydroxyl protective reagent to a hydroxyl protection reaction to give compound (3); preferably, the feeding molar ratio of compound (2) to the hydroxyl protective reagent is 1:(1-3); more preferably, the feeding molar ratio of compound (2) to the hydroxyl protective reagent is 1:(1-2); even more preferably, the feeding molar ratio of compound (2) to the hydroxyl protective reagent is 1:(1.2-1.5); most preferably, the feeding molar ratio of compound (2) to the hydroxyl protective reagent is 1:1.3;
step b': reacting compound (3) with acid HY to give compound (4a); preferably, the acid HY is hydrochloric acid, hydrobromic acid, formic acid, hydroiodic acid, *p*-toluenesulfonic acid, or trifluoromethanesulfonic acid;
step c': reacting compound (4a) under an action of a base to give compound (4); preferably, the base is sodium hydroxide, potassium hydroxide, sodium carbonate, or potassium carbonate;
step d': subjecting compound (4) and an amino protective reagent to an amino protection reaction to give compound (5); preferably, the feeding molar ratio of compound (4) to the amino protective reagent is 1:1-1.5; and
step e': reacting compound (5) in a fourth solvent under an action of a basic reagent to give compound (6); preferably, the basic reagent is sodium hydride, sodium methoxide, sodium hydroxide, potassium hydroxide, potassium *tert*-butoxide, sodium *tert*-butoxide, potassium carbonate, sodium carbonate, or sodium ethoxide; preferably, the feeding molar ratio of compound (5) to the basic reagent is 1:(1-3), more preferably 1:(1.5-2.5), and most preferably 1:2.2; preferably, the fourth solvent is tetrahydrofuran or *N*,*N*-dimethylformamide, or a combination thereof; preferably, the reaction temperature is -10 to 50 °C; further preferably, the reaction temperature is -10 to 15 °C; even more preferably, the reaction temperature is -5 to 10 °C; most preferably, the reaction temperature is 0-10 °C.

In some embodiments, in step a of the process of the present disclosure, compound (6) is prepared by the following steps: 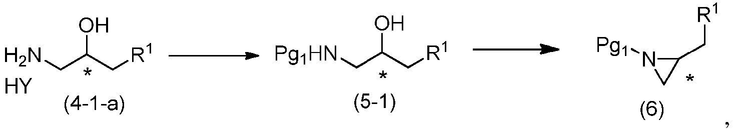
wherein "*" on compound (4-1-a) and compound (5-1) represents that compound (4-1-a) and compound (5-1) are both in S configuration, and "*" on compound (6) represents that compound (6) is in R configuration; or "*" on compound (4-1-a) and compound (5-1) represents that compound (4-1-a) and compound (5-1) are both in R configuration, and "*" on compound (6) represents that compound (6) is in S configuration;
step a": subjecting compound (4-1-a) and an amino protective reagent to an amino protection reaction under an action of a base to give compound (5-1); preferably, the feeding molar ratio of compound (4-1-a) to the amino protective reagent is 1:(0.7-2); preferably, the base is sodium hydroxide, potassium hydroxide, sodium carbonate, or potassium carbonate; and
step b": reacting compound (5-1) at a low temperature under an action of triphenylphosphine and ethyl azodicarboxylate and under nitrogen atmosphere to give compound (6), wherein the low temperature is -40 to 0 °C, preferably -30 to -10 °C, and more preferably -20 to -10 °C.

In some embodiments, in step a of the process of the present disclosure, compound (6) is prepared by the following steps: 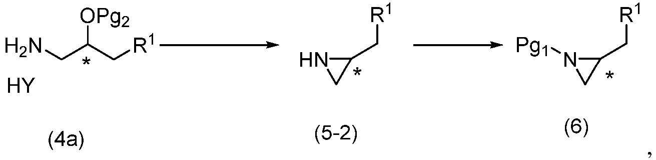 wherein
"*" on compound (4a) represents that compound (4a) is in S configuration, and "*" on compound (5-2) and compound (6) represents that compound (5-2) and compound (6) are both in R configuration; or "*" on compound (4a) represents that compound (4a) is in R configuration, and "*" on compound (5-2) and compound (6) represents that compound (5-2) and compound (6) are both in S configuration;
step a‴: reacting compound (4a) under an action of a base to give compound (5-2); preferably, the base is sodium hydroxide, potassium hydroxide, sodium carbonate, or potassium carbonate; preferably, the feeding molar ratio of compound (4a) to the base is 1:(1-3), more preferably 1:(1.2-2.5), and most preferably 1:1.5; preferably, the reaction temperature is 10-100 °C; more preferably, the reaction temperature is 30-80 °C; even more preferably, the reaction temperature is 40-60 °C; most preferably, the reaction temperature is 50 °C; and
step b‴: continuously subjecting compound (5-2) and an amino protective reagent to an amino protection reaction to give compound (6); preferably, the feeding molar ratio of compound (4-1-a) to the amino protective reagent is 1:(0.7-2); preferably, the reaction is a low-temperature reaction, and the low temperature is -5 to 15 °C; more preferably, the low temperature is 0-10 °C.

In another aspect, the present disclosure provides a compound having a structure of formula (7a) or (7b) below: 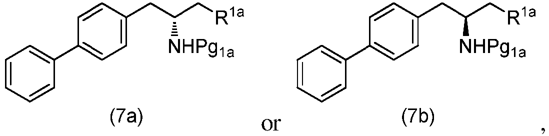
wherein R^{1a} is F, Cl, Br, I, or -OR³, and R³ represents C₁₋₆ alkyl or C₁₋₆ alkyl containing a heteroatom substitution;
Pg₁ₐ is an amino protective group, preferably benzyloxycarbonyl, *tert*-butoxycarbonyl, methoxycarbonyl, ethoxycarbonyl, isopropyloxycarbonyl, isobutyloxycarbonyl, fluorenylmethoxycarbonyl, allyloxycarbonyl, or trimethylsilylethoxycarbonyl;
and when R^{1a} is Br or I, Pg₁ₐ is not *tert*-butoxycarbonyl.

In some embodiments, compound (7a) or compound (7b) of the present disclosure has one of the following structures:

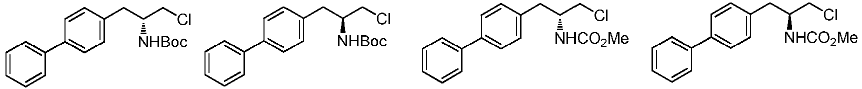

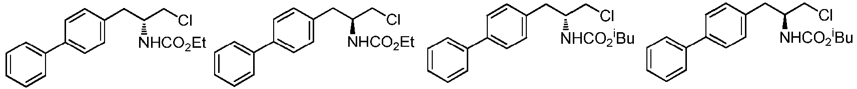

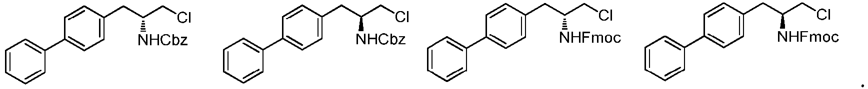

In another aspect, the present disclosure provides a compound having a structure of formula (6a) or (6b) below: 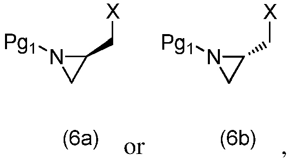
wherein X is F, Cl, Br, or I; and
Pg₁ is an amino protective group, preferably benzyloxycarbonyl, *tert*-butoxycarbonyl, methoxycarbonyl, ethoxycarbonyl, isopropyloxycarbonyl, isobutyloxycarbonyl, fluorenylmethoxycarbonyl, allyloxycarbonyl, or trimethylsilylethoxycarbonyl.

In some embodiments, compound (6a) or compound (6b) of the present disclosure has one of the following structures:

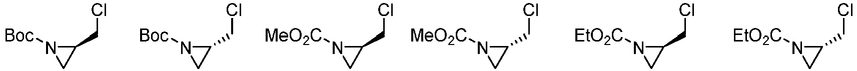

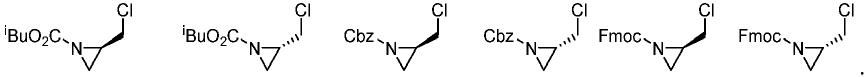

In another aspect, the present disclosure provides a compound having a structure of formula (8-a) or (8-b) below:

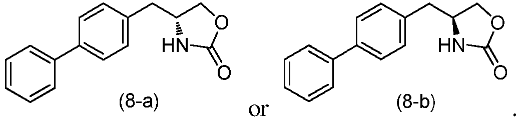

In another aspect, the present disclosure provides use of the process according to the present disclosure or the compound according to the present disclosure in the preparation of sacubitril.

### Beneficial Effects

Compared with the currently reported routes, the route of the present application has the advantages of high yield, simple and convenient operation, realization of continuous operation, ease of scale-up for production, low price and easy obtainment of used starting materials, low costs, and the like. Specifically:
1. The auxiliary materials used in the process route of the present application, such as sodium hydroxide, hydrochloric acid, methanesulfonyl chloride, and the like, are common materials for large-scale industrial production. The auxiliary materials are cheap and easily available, and have excellent economical efficiency, and low production costs.
2. In the process route of the present application, a plurality of intermediates can be subjected to continuous feeding production without separation and purification, and the energy consumption and labor costs of industrial production can be effectively reduced. For example, Example 2 can be directly performed after the solvent is recovered under reduced pressure in Example 1, the product obtained by directly performing salt forming operation on the organic phase separated in step 2 of Example 1 enters the aqueous phase and proceeds directly to Example 3. In step 6 of Example 7, a three-step reaction is included, using toluene as the solvent, which allows for a one-pot process, and simultaneously achieves the purification of the product (10-a).

### Detailed description

Generally, the compounds of the present disclosure may be prepared by the methods described in the present disclosure. The following reaction schemes and examples serve to further illustrate the context of the present disclosure. It should be understood by those skilled in the art that the examples are only intended to help understand the present disclosure and should not be construed as the specific limitation of the present disclosure.

Unless otherwise indicated, all temperatures in the examples described below are given in Celsius degrees. The room temperature of the present disclosure is 10-30 °C or 15-25 °C. Unless otherwise stated, reagents are conventional and commercially available. The chromatographic column is a silica gel column. Nuclear magnetic resonance spectroscopy is performed using CDCl₃ or DMSO-*d₆* as the solvent (reported in ppm) and TMS (0 ppm) or chloroform (7.25 ppm) as the reference standard. Coupling constants *J* are expressed in hertz (Hz).

The following abbreviations are used throughout the present disclosure:

| | | | |
|---|---|---|---|
| Benzyloxycarbonyl | -Cbz | *tert*-Butoxycarbonyl | -Boc |
| Methoxycarbonyl | -COOMe or -CO₂Me | Ethoxycarbonyl | -COOEt or -CO₂Et |
| Isobutyloxycarbonyl | -COO*ⁱ*Bu or -CO₂*ⁱ*Bu | Fluorenylmethoxycarbonyl | -Fmoc |
| Isopropyloxycarbonyl | -COO*ⁱ*Pr or -CO₂*ⁱ*Pr | Trimethylsilylethoxycarbonyl | -Teoc |
| Allyloxycarbonyl | -Alloc | Trifluoromethanesulfonyl | -OTf |
| Methanesulfonyl | -OMs | Nitrosulfonyl | -Ns |
| *p*-Toluenesulfonyl | -Ts | Ethyl acetate | EA, EtOAc |
| Petroleum ether | PE | Tetrahydrofuran | THF |
| Chloroform-*d* | deuterated chloroform | DMSO-*d₆* | deuterated dimethyl sulfoxide |
| M, mol/L | mol/liter | | |

### Examples

### Example 1: Synthesis of Compound (6-a)

### Step 1: synthesis of compound (2-a)

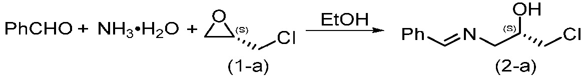

Benzaldehyde (50 g, 0.47 mol) was added to ethanol (300 mL), and the mixture was stirred at room temperature. Aqueous ammonia (25*%*, 50 mL) was dropwise added to the reaction system at 15-20 °C. After the dropwise addition was completed, the mixture was warmed to room temperature and stirred for 20 min, and then (*S*)-epichlorohydrin (52 g, 0.56 mol, 1.2 eq) was diluted with ethanol (50 mL) and slowly and dropwise added to the reaction system under nitrogen atmosphere. After the dropwise addition was completed, the system was stirred at room temperature (25-30 °C) for 12 h. After epichlorohydrin disappeared as monitored by GC, the reaction was completed. Ethanol was recovered by distillation under reduced pressure to give a yellow oily product, and ethanol (100 mL) was added for distillation again to give a pale yellow oily liquid (113 g), which was crude compound (2-a).

Screening of different process conditions in step 1 of Example 1: According to the reagent feeding amounts shown in Table 1, the rest of operations were performed as described in step 1 to give compound (2-a). A GC content of the compound (2-a) obtained was detected, and the results are shown in Table 1.

**Table 1. Screening of different process conditions in step 1 of Example 1**

| | Benzaldehyde | Epichlorohydrin | Aqueous ammonia (25%) | Ethanol | Nitrogen | GC content |
|---|---|---|---|---|---|---|
| 01 | 18.7g | 24 g (1.5eq) | 14.7 g | 50 mL | Absent | 79 *%* |
| 02 | 18.7g | 24 g (1.5eq) | 14.7 g | 50 mL | Present | 92 *%* |
| 03 | 18.7g | 24 g (1.2eq) | 14.7 g | 50 mL | Present | 91 *%* |
| 04 | 100 g | 104 g (1.2eq) | 120 g | 400 mL | Present | 94*%* |

The screening and optimizing of the reaction conditions show that, with all other conditions being identical to the paragraph immediately preceding Table 1, use of nitrogen protection during the dropwise addition of epichlorohydrin can effectively reduce the formation of impurities and increase the yield. Simultaneously, the reaction scale-up yielded similar results to the small-scale experiment, indicating good reaction stability.

### Step 2: synthesis of compound (4-a)

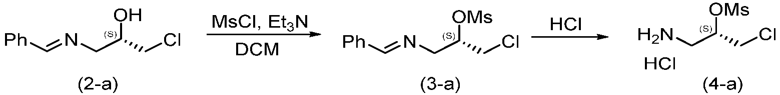

The crude compound (2-a) (113 g, 0.42 mol, 1.0 eq) obtained in the previous step (step 1) was added to dichloromethane (400 mL), and the mixture was stirred at room temperature until a small amount of a white insoluble substance was observed. The mixture was filtered, and triethylamine (63.7 g, 0.629 mol, 1.5 eq) was added to the filtrate. The mixture was cooled to an internal temperature of 0-10 °C, and methanesulfonyl chloride (62.5 g, 0.545 mol, 1.3 eq) diluted with dichloromethane (100 mL) was dropwise added. The system was heated to an internal temperature of 12 °C, and a white solid precipitated. After the dropwise addition was completed, the system was cooled to 4 °C and stirred for 1 h with the temperature maintained. Then the starting materials disappeared as monitored by normal phase HPLC. Water (400 mL) was added to the system, and the mixture was uniformly stirred for 30 min and extracted. The organic phase was washed twice with water (400 mL × 2). Concentrated hydrochloric acid (120 g) diluted with water (240 mL) was added to the organic phase. The mixture was uniformly stirred at room temperature for 3-4 h and left to stand for liquid separation. The organic phase was washed with water (50 mL), and the aqueous phases were combined to give an aqueous solution of crude compound (4-a), which was directly used for the subsequent reaction.

Screening of different process conditions in step 2 of Example 1: According to the reagent feeding amounts shown in Table 2, the rest of operations were performed as described in step 2 of Example 1 to give an aqueous solution of compound (4-a). Conversion rates of compound (2-a) were detected, and the results are shown in Table 2.

**Table 2. Screening of different process conditions in step 2 of Example 1**

| | 2-a (crude product) | Methanesulfonyl | Triethylamine | Dichloromethane | Conversion |
|---|---|---|---|---|---|
| | | chloride | | | rate |
| 01 | 113 g (0.4mol) | 67 g (1.4eq) | 63.7 g (1.5eq) | 500 mL | 100 *%* |
| 02 | 113 g (0.4mol) | 62 g (1.3eq) | 63.7 g (1.5eq) | 500 mL | 100 *%* |
| 03 | 113 g (0.4mol) | 57 g (1.2eq) | 63.7 g (1.5eq) | 500 mL | 98 *%* |
| 04 | 113 g (0.4mol) | 48 g (leq) | 63.7 g (1.5eq) | 500 mL | 80 *%* |

Under reaction conditions similar to those in the paragraph immediately preceding Table 2, it was found that the conversion rate of the hydroxyl protection reaction for the same batch of (2-a) was related to the amount of methanesulfonyl chloride used. When using an equivalent of 1.3 eq, the reaction can be completely converted.

### Step 3: synthesis of compound (5-a)

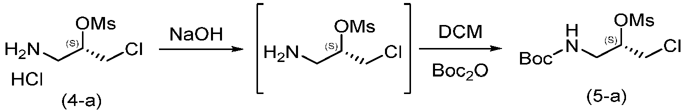

The aqueous solution of the crude compound (4-a) (0.374 mol, 1.0 eq) of step 2 was added to a 2 L reaction flask and cooled to an internal temperature of 0-10 °C, and then an aqueous sodium hydroxide solution (sodium hydroxide (17 g) dissolved in water (100 mL), 1.1 eq) was dropwise added to adjust the pH to 8-9. The system gradually turned into a white turbid state, and after the dropwise addition was completed, the system was maintained at 0-10 °C. Di-*tert*-butyl dicarbonate (82 g, 1 eq) diluted with dichloromethane (100 mL) was dropwise added to the reaction system. The system generated gas and released heat, and the system was heated to an internal temperature of 10-15 °C. After the dropwise addition was completed, the mixture was left to react for 2 h with the temperature maintained. After the starting materials disappeared as monitored by LC-MS, the reaction was completed. The mixture was left to stand for liquid separation, and dichloromethane (137 g) was added to the aqueous phase for extraction. The organic phases were separated, combined, and washed with water. The organic phase was dried, filtered, and concentrated under reduced pressure to give a pale yellow oily liquid, which became a white waxy solid (126 g) after standing, which was crude compound (5-a).

Characterization data for (5-a): ¹H NMR (600 MHz, Chloroform-*d*) δ 5.09 (s, 1H), 4.89 (s, 1H), 3.81-3.76 (m, 1H), 3.70 (dd, J = 12.4, 6.1 Hz, 1H), 3.55 (dq, J = 10.2, 5.2 Hz, 1H), 3.45 (dt, J = 14.2, 6.2 Hz, 1H), 3.13 (s, 3H), 1.45 (s, 9H). ¹³C NMR (151 MHz, CDCl₃) δ 155.92, 80.16, 79.85, 43.58, 42.36, 38.38, 28.22.

Screening of different process conditions in step 3 of Example 1: According to the reagent feeding amounts shown in Table 3, the rest of operations were performed as described in step 3 of Example 1 to give crude compound (5-a). Conversion rates of the compound (4-a) were detected, and the results are shown in Table 3.

**Table 3. Screening of different process conditions in step 3 of Example 1**

| | 4-a (crude product) | Sodium hydroxide | Di-*tert*-butyl | Dichloromethane | Conversion |
|---|---|---|---|---|---|
| | | | dicarbonate | | rate |
| 01 | Aqueous solution (0.34 mol) | 15 g (1.1eq) | 74 g (1 eq) | 500 mL | 100 % |
| 02 | Aqueous solution (0.34 mol) | 15 g (1.1eq) | 140 g (1.5eq) | 500 mL | 100 % |

Under reaction conditions similar to those in the paragraph immediately preceding Table 3, when the amount of di-*tert*-butyl dicarbonate used was 1-1.5 eq, the reactions can all be completely converted.

### Step 4: synthesis of compound (6-a)

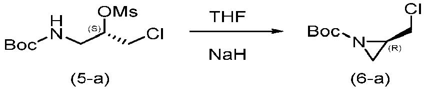

The crude compound (5-a) (126 g) obtained in step 3 was added to tetrahydrofuran (500 mL) and completely dissolved by stirring at room temperature. The mixture was cooled to 0-10 °C, and a sodium hydride solid (60*%*, 20 g, 0.83 mol, 2 eq) was added to the reaction. The system generated gas foam, and was warmed to room temperature (25-30 °C) and left to react for 1 h. Then the starting materials disappeared as monitored by LC-MS. Tetrahydrofuran was recovered under reduced pressure. Dichloromethane and water were added to the residue for extraction. The organic phase was dried and precipitated to give a pale yellow oily liquid (85 g), which was purified by distillation under reduced pressure to give a colorless oily liquid (51.6 g) as compound (6-a) with a GC purity of 92*%*, and a total yield from the reaction of the compound PhCHO in step 1 to the final synthesis of the compound (6-a) in step 4 was 58.3*%*.

Characterization data for (6-a): ¹H NMR (600 MHz, Chloroform-d) δ 3.63 (dd, J = 10.0, 4.1 Hz, 1H), 3.46 (dd, J = 11.3, 5.9 Hz, 1H), 2.78 - 2.70 (m, 1H), 2.38 (d, J = 4.0 Hz, 1H), 2.12 (s, 1H), 1.46 (s, 9H). ¹³C NMR (151 MHz, CDCl₃) δ 160.96, 81.15, 44.50, 37.12, 30.92, 27.43.

Screening of different process conditions in step 4 of Example 1: According to the reagent feeding amounts shown in Table 4, the rest of operations were performed as described in step 4 of Example 1 to give compound (6-a). The yield results are shown in Table 4.

**Table 4. Screening of different process conditions in step 4 of Example 1**

| | 5-a | Base (2 eq) | Solvent | A total yield from the reaction of the compound PhCHO to the final synthesis of the compound (6-a) |
|---|---|---|---|---|
| 01 | 500 mg | NaOH | DCM | <30*%* |
| 02 | 500 mg | K₂CO₃ | DCM | 0*%* |
| 03 | 500 mg | t-BuOK | DCM | <30*%* |
| 04 | 500 mg | Cs₂CO₃ | DCM | 30∼40*%* |
| 05 | 500 mg | NaOH | EtOH | 0*%* |
| 06 | 500 mg | t-BuOK | EtOH | <10*%* |
| 07 | 500 mg | NaOH | THF | 50∼60*%* |
| 08 | 500 mg | t-BuOK | THF | <30*%* |
| 09 | 5 g | NaH | THF | 80∼90*%* |
| 10 | 5 g | NaH | DCM | <10*%* |
| 11 | 5 g | NaH | DMF | 70∼80*%* |
| 12 | 20 g | NaH | THF | 84*%* |

Under reaction conditions similar to those in the paragraph immediately preceding Table 4, the reaction yield was the highest when NaH was used as the base and THF was used as the solvent.

### Example 2: Synthesis of Compound (6-a)

### Step 1: synthesis of compound (5-1-a)

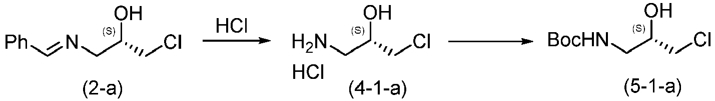

The crude product (2-a) (113 g, 0.4 mol, 1.0 eq) prepared in step 1 of Example 1 was added to dichloromethane (200 mL) serving as a solvent. Concentrated hydrochloric acid (86 g, 1.0 eq) diluted with water (100 mL) was added dropwise to the reaction. After the dropwise addition was completed, the mixture was uniformly stirred for 1 h at room temperature (25-30°C) and left to stand for phase separation. The organic phase was washed with water (50 mL) and then the aqueous phases were combined. The aqueous phase was cooled to an internal temperature of 0-10 °C, and sodium hydroxide (17 g) dissolved in water (50 mL) was slowly and dropwise added to the reaction. After the dropwise addition was completed, di-*tert*-butyl dicarbonate (65 g, 0.417 mol, 1 eq) diluted with dichloromethane (100 mL) was slowly and dropwise added to the reaction system. The system released heat and generated gas. After the dropwise addition was completed, the mixture was warmed to room temperature and stirred for 2 h. Then the starting materials were completely converted as detected by LC-MS. The mixture was left to stand for phase separation, the aqueous phase was washed with dichloromethane (100 mL), and the organic phases were combined, dried, filtered, and precipitated under reduced pressure to give a white solid (98 g), which was crude compound (5-1-a).

### Step 2: synthesis of compound (6-a)

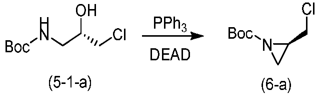

The crude compound (5-1-a) (98 g, 0.376 mol, 1 eq) obtained in the previous step (step 1 of Example 2) was added to toluene (300 mL) and completely dissolved by stirring at room temperature. Triphenylphosphine (108 g, 0.413 mol, 1.1 eq) was added under nitrogen atmosphere, and the mixture was cooled to an internal temperature of -10 to -20 °C. Diethyl azodicarboxylate (68.7 g, 0.394 mol, 1.05 eq) was slowly and dropwise added to the reaction system. The system gradually changed from colorless to yellow, and a solid precipitated. After the dropwise addition was completed, the mixture was left to react for 2 h with the temperature maintained, and the starting materials disappeared as monitored by LC-MS. For the post-treatment, water (150 mL) was added to the reaction, and the mixture was stirred at room temperature and left to stand for phase separation. The organic phase was precipitated under reduced pressure to give a yellow oily liquid (115 g), which was purified by distillation under reduced pressure to give a colorless oily liquid (64 g), which was pure compound (6-a). The GC purity was 93%. A total yield of the reactions from step 1 of Example 1 to the final synthesis of the compound (6-a) of Example 2 was calculated to be 73*%*.

### Example 3: Synthesis of Compound (6-a)

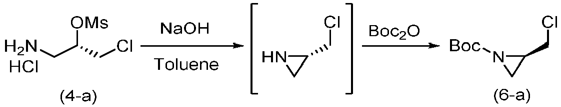

The aqueous solution of the crude compound (4-a) obtained in step 2 of Example 1 (0.356 mol, 1 eq) was cooled to 5-10°C. Sodium hydroxide (15 g, 0.392 mol, 1.1 eq) diluted with water (50 mL) was slowly and dropwise added to the reaction system. After the dropwise addition was completed, the system was supplemented with toluene (200 mL), heated to 50°C, and left to react for 1-2 h. Then the starting materials were completely converted as monitored by LC-MS. The system was cooled to 0-10°C, and di-*tert*-butyl dicarbonate (71.5 g, 0.327 mol, 1 eq) was dropwise added to the reaction system. After the dropwise addition was completed, the system was warmed to room temperature and left to react for 2-3 h, and then LC-MS indicated the disappearance of the starting materials and the generation of the desired product. The mixture was left to stand for liquid separation, and the aqueous phase was washed with toluene (100 mL). Then the organic phases were combined and precipitated under reduced pressure to give a yellow oil (85 g). The compound (6-a) was obtained as a colorless oily liquid (60 g) by purifying by distillation under reduced pressure with a GC purity of 95%; and a total yield of the reactions from step 1 of Example 1 to the final synthesis of the compound (6-a) of Example 3 was calculated to be 68*%*.

### Example 4: Synthesis of Compound (10-a)

### Step 1: synthesis of compound (7-a)

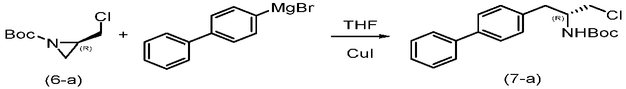

The compound (6-a) (51.6 g, 0.27 mol, 1.0 eq) obtained in step 4 of Example 1 was dissolved in tetrahydrofuran (200 mL) to give a solution of the compound (6-a) in tetrahydrofuran for later use. Copper(I) iodide (0.5 g, 0.003 mol, 0.01 eq) was added to a dry and oxygen-free reaction flask, and biphenylmagnesium bromide (134 mL, 2.0 M, 1.0 eq) was added to the reaction system under nitrogen atmosphere. The system was cooled to an internal temperature of -15 to -20 °C, and then the solution of the compound (6-a) in tetrahydrofuran was dropwise added to the reaction system. The system gradually changed to green. After the dropwise addition was completed, the mixture was left to react for 1 h with the temperature maintained at -5 to -10 °C. GC monitoring indicated that the reaction was completed, and the reaction was quenched by adding water (50 mL). Tetrahydrofuran was recovered under reduced pressure. Then dichloromethane (300 mL) and water (400 mL) were added for extraction. Liquid separation was performed, and the organic phase was dried and precipitated to give a white flocculent solid, which was slurried with *n*-heptane to give pure compound (7-a) (74 g) with a yield of 79*%*.

Characterization data for (7-a): ¹H NMR (600 MHz, Chloroform-*d*) δ 7.56 (dd, J = 19.0, 7.5 Hz, 4H), 7.43 (t, J = 7.4 Hz, 2H), 7.33 (dd, J = 15.2, 7.4 Hz, 3H), 4.85 (d, J = 6.8 Hz, 1H), 4.17 (s, 1H), 3.65 (d, J = 10.0 Hz, 1H), 3.54 (d, J = 11.1 Hz, 1H), 2.94 (dt, J = 21.5, 8.7 Hz, 2H), 1.44 (s, 9H). ¹³C NMR (151 MHz, CDCl₃) δ 155.03, 140.75, 139.72, 136.13, 129.71, 128.76, 127.36, 127.23, 126.98, 79.84, 51.98, 46.96, 37.41, 28.33.

Screening of different process conditions in step 1 of Example 4: According to the reagent feeding amounts shown in Table 5, the rest of operations were performed as described in step 1 of Example 4 to give compound (7-a). The results are shown in Table 5.

**Table 5. Screening of different process conditions in step 1 of Example 4**

| | 6-a | Biphenylmagnesium bromide | Copper(I) iodide | THF | Reaction temperature |
|---|---|---|---|---|---|
| 01 | 4 g | 11mL(2.0M) | 0.3 g (0.1eq) | 20 mL | -10 °C→40 °C |
| 02 | 4 g | 11 mL(2.0M) | 0.3 g (0.1eq) | 20 mL | -10 °C |
| 03 | 30 g | 75 mL(2.0M) | 220 mg (0.01eq) | 150 mL | -10~-5°C |

The price of copper(I) iodide was high, researches show that the copper(I) iodide can still effectively catalyze the reaction when the using amount was 0.01 eq, and the reaction effect was almost the same as that of the small-scale experiment when the reaction was preliminarily scaled up.

### Step 2: synthesis of compound (10-a)

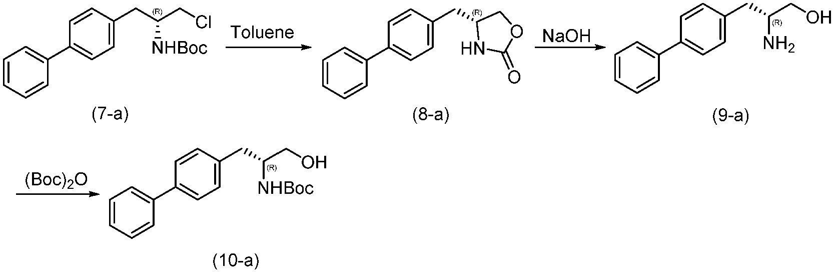

Compound (7-a) (70 g, 0.2 mol, 1 eq) was added to toluene (200 mL). The mixture was heated to 110 °C at reflux for 0.5 h, and then (7-a) disappeared, and compound (8-a) was generated as monitored by LC-MS.

Characterization data for compound (8-a): ¹H NMR (400 MHz, DMSO-*d₆*) δ 7.83 (s, 1H), 7.69-7.64 (m, 2H), 7.61 (d, J = 8.2 Hz, 2H), 7.46 (t, J = 7.6 Hz, 2H), 7.40-7.31 (m, 3H), 4.30 (t, J = 8.2 Hz, 1H), 4.15-4.05 (m, 1H), 4.03 (dd, J = 8.2, 5.4 Hz, 1H), 2.84 (qd, J = 13.6, 6.1 Hz, 2H); ¹³C NMR (101 MHz, DMSO) δ 159.11, 140.35, 138.87, 136.34, 130.49, 129.40, 127.79, 127.14, 127.00, 68.57, 52.95, 40.35.

Sodium hydroxide (24 g, 0.6 mol, 3 eq) was added to the reaction, and the mixture was left to react for 3 h with the temperature maintained at 80 °C. Then compound (8-a) disappeared, and compound (9-a) was generated as monitored by LC-MS.

The system was cooled to 25-30 °C, and after the addition of water (100 mL), di-*tert-*butyl dicarbonate (44 g, 0.2 mol, 1 eq) was dropwise added. The system gradually became turbid, and a white solid precipitated. After the dropwise addition was completed, the mixture was left to react for 2 h with the temperature maintained. HPLC indicated the disappearance of compound (9-a) and the generation of compound (10-a). The system was supplemented with toluene (200 mL), heated to 60 °C, stirred for 30 min, and left to stand for liquid separation with the temperature maintained. The aqueous phase was discarded, and the organic phase was slowly cooled to 0 °C to precipitate a white solid. The solid was filtered and dried to give compound (10-a) (white solid) (53 g) with a purity of 99.5*%*. A total yield for the three steps was 85.5*%*.

### Example 5: Synthesis of Compound (8-a)

### Step 1: synthesis of compound (5-2-a)

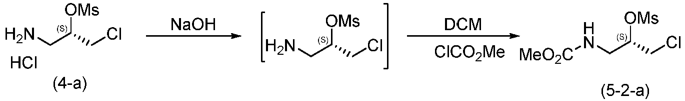

The aqueous solution of the crude compound (4-a) (0.374 mol, 1.0 eq) obtained in step 2 of Example 1 was added to a 2 L reaction flask and cooled to an internal temperature lower than 10 °C, and then an aqueous sodium hydroxide solution (sodium hydroxide (17 g) dissolved in water (100 mL), 1.1 eq) was dropwise added to adjust the pH to 8-9. The system gradually turned into a white turbid state, and after the dropwise addition was completed, the system was cooled to 0-10 °C. Methyl chloroformate (37 g, 1.1 eq) diluted with dichloromethane (100 mL) was dropwise added to the reaction system. The system generated gas and released heat, and the system was heated to an internal temperature of 10-15 °C. After the dropwise addition was completed, the mixture was left to react for 2 h with the temperature maintained. After the starting materials disappeared as monitored by LC-MS, the reaction was completed. The mixture was left to stand for liquid separation, and dichloromethane (137 g) was added to the aqueous phase for extraction. The organic phases were separated, combined, and washed with water. The organic phase was dried, filtered, and concentrated under reduced pressure to give a pale yellow oily liquid, which became a white waxy solid (105 g) after standing, which was crude compound (5-2-a).

Characterization data for compound (5-2-a): ¹H NMR (600 MHz, Chloroform-*d*) δ 5.79 (s, 1H), 4.89 (s, 1H), 3.81 (d, J = 11.6 Hz, 1H), 3.73 (dd, J = 11.9, 6.0 Hz, 1H), 3.68 (s, 3H), 3.58 (d, J = 14.4 Hz, 1H), 3.54-3.47 (m, 1H), 3.14 (s, 3H); ¹³C NMR (151 MHz, CDCl₃) δ 157.12, 79.40, 52.15, 43.36, 42.30, 38.04.

### Step 2: synthesis of compound (6-2-a)

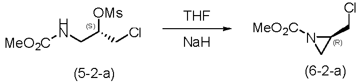

The crude compound (5-2-a) (105 g, 0.32 mol, 1 eq) obtained in step 2 of Example 5 was added to tetrahydrofuran (400 mL), and the compound was completely dissolved by stirring at room temperature. The mixture was cooled to 0-10 °C, and a sodium hydride solid (60*%*, 19.2 g, 0.48 mol, 1.5 eq) was added to the reaction. The system generated gas foam, and was warmed to room temperature (25-30 °C) and left to react for 1 h. Then the starting materials disappeared as monitored by LC-MS. Tetrahydrofuran was recovered under reduced pressure. Then dichloromethane and water were added for extraction. The organic phase was dried and precipitated to give a pale yellow oily liquid (58 g). The colorless oily liquid (43.5 g) was obtained as compound (6-2-a) by purifying by distillation under reduced pressure with a GC purity of 95*%*, and a total yield of the first five steps of Example 1 was calculated to be 62.5*%*.

Characterization data for compound (6-2-a): ¹H NMR (600 MHz, Chloroform-*d*) δ 3.74 (s, 3H), 3.65 (dd, J = 11.6, 6.0 Hz, 1H), 3.51 (dd, J = 11.6, 5.7 Hz, 1H), 2.82 (dt, J = 5.4, 2.5 Hz, 1H), 2.47 (d, J = 6.0 Hz, 1H), 2.20 (s, 1H). ¹³C NMR (151 MHz, CDCl₃) δ 162.60, 53.39, 44.35, 37.17, 30.93.

### Step 3: synthesis of compound (7-2-a)

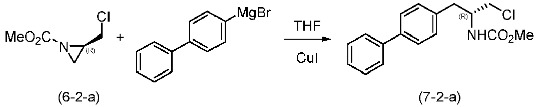

The compound (6-2-a) (43 g, 0.287 mol, 1.0 eq) obtained in step 2 was dissolved in tetrahydrofuran (200 mL) to give a solution of the compound (6-a) in tetrahydrofuran for later use.

Copper(I) iodide (0.5 g, 0.0028 mol, 0.01 eq) was added to a dry and oxygen-free reaction flask, and biphenylmagnesium bromide (158 mL, 2.0 M, 1.1 eq) was added to the reaction system under nitrogen atmosphere. The system was cooled to an internal temperature of -15 to -20 °C, and then the solution of the compound (6-a) in tetrahydrofuran was dropwise added to the reaction system. After the dropwise addition was completed, the mixture was left to react for 1 h with the temperature maintained at -10 to -5 °C. GC monitoring indicated that the reaction was completed, and the reaction was quenched by adding water (50 mL). Tetrahydrofuran was recovered under reduced pressure. Then dichloromethane (300 mL) and water (400 mL) were added for extraction. Liquid separation was performed, and the organic phase was dried and precipitated to give a white flocculent solid, which was slurried with n-heptane to give pure compound (7-2-a) (70 g) with a yield of 80*%*.

Characterization data for compound (7-2-a): ¹H NMR (400 MHz, Chloroform-*d*) δ 7.60-7.51 (m, 4H), 7.43 (t, J = 7.6 Hz, 2H), 7.37-7.27 (m, 3H), 5.03 (d, J = 7.4 Hz, 1H), 4.22 (s, 1H), 3.68 (s, 3H), 3.64 (d, J = 3.5 Hz, 1H), 3.54 (dd, J = 11.2, 3.2 Hz, 1H), 2.96 (t, J = 6.4 Hz, 2H); ¹³C NMR (101 MHz, CDCl₃) δ 156.19, 140.63, 139.81, 135.82, 129.63, 128.75, 127.40, 127.25, 126.95, 52.48, 52.24, 46.71, 37.30.

### Step 4: synthesis of compound (8-a)

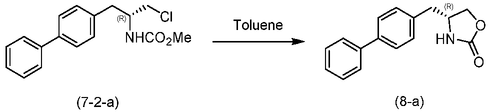

The compound (7-2-a) (70 g) obtained in step 3 was added to a 500 mL reaction flask, and toluene (200 mL) was added. The mixture was heated to 110 °C and stirred at reflux for 2-3 h. Compound (7-2-a) disappeared, and compound (8-a) was generated as monitored by LC-MS. Toluene was recovered under reduced pressure to give a pale yellow solid (59.5 g), which could be directly used for the subsequent reaction.

### Example 6: Synthesis of Compound (8-a)

### Step 1: synthesis of compound (5-3-a)

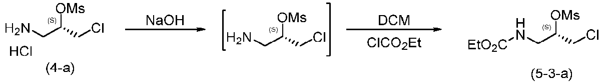

The aqueous solution of the crude compound (4-a) (0.374 mol, 1.0 eq) of step 2 of Example 1 was added to a 2 L reaction flask and cooled to an internal temperature lower than 10 °C, and then an aqueous sodium hydroxide solution (sodium hydroxide (17 g) dissolved in water (100 mL), 1.1 eq) was dropwise added to adjust the pH to 8-9. The system gradually turned into a white turbid state, and after the dropwise addition was completed, the system was cooled to 0-10 °C. Ethyl chloroformate (43.9 g, 1.1 eq) diluted with dichloromethane (100 mL) was dropwise added to the reaction system. The system generated gas and released heat, and the system was heated to an internal temperature of 10-15 °C. After the dropwise addition was completed, the mixture was left to react for 2 h with the temperature maintained. After the starting materials disappeared as monitored by LC-MS, the reaction was completed. The mixture was left to stand for liquid separation, and dichloromethane was added to the aqueous phase for extraction. The organic phases were separated and combined. The organic phase was dried, filtered, and concentrated under reduced pressure to give a pale yellow oily liquid, which became a white waxy solid (112 g) after standing, which was crude compound (5-3-a).

Characterization data for compound (5-3-a): ¹H NMR (600 MHz, Chloroform-*d*) δ 5.44 (s, 1H), 4.90 (s, 1H), 4.13 (d, J = 6.7 Hz, 2H), 3.80 (d, J = 12.2 Hz, 1H), 3.72 (dd, J = 12.2, 6.0 Hz, 1H), 3.60 (d, J = 14.5 Hz, 1H), 3.51 (dt, J = 13.7, 6.0 Hz, 1H), 3.13 (s, 3H), 1.25 (t, J = 6.4 Hz, 3H); ¹³C NMR (151 MHz, CDCl₃) δ 156.76, 79.63, 61.21, 43.47, 42.50, 38.28, 14.39.

### Step 2: synthesis of compound (6-3-a)

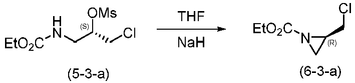

The crude compound (5-3-a) (112 g, 0.32 mol, 1 eq) of step 3 was added to tetrahydrofuran (400 mL), and the compound was completely dissolved by stirring at room temperature. The mixture was cooled to 0-10 °C, and a sodium hydride solid (60*%*, 26.18 g, 0.65 mol, 2 eq) was added to the reaction. The system generated gas foam, and was warmed to room temperature (25-30 °C) and left to react for 1 h. Then the starting materials disappeared as monitored by LC-MS. Tetrahydrofuran was recovered under reduced pressure. Then dichloromethane and water were added for extraction. The organic phase was dried and precipitated to give a pale yellow oily liquid (54 g). The colorless oily liquid (47.6 g) was obtained as compound (6-3-a) by purifying by distillation under reduced pressure with a GC purity of 94*%*, and a total yield of the first five steps of Example 1 was calculated to be 63*%*.

### Step 3: synthesis of compound (7-3-a)

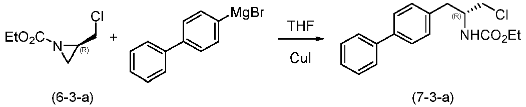

The compound (6-3-a) (47 g, 0.287 mol, 1.0 eq) obtained in step 2 was dissolved in tetrahydrofuran (200 mL) to give a solution of the compound (6-3-a) in tetrahydrofuran for later use.

Copper(I) iodide (0.5 g, 0.0028 mol, 0.01 eq) was added to a dry and oxygen-free reaction flask, and biphenylmagnesium bromide (158 mL, 2.0 M, 1.1 eq) was added to the reaction system under nitrogen atmosphere. The system was cooled to an internal temperature of -15 to -20 °C, and then the solution of the compound (6-3-a) in tetrahydrofuran was dropwise added to the reaction system. After the dropwise addition was completed, the mixture was left to react for 1 h with the temperature maintained at -5 to -10 °C. GC monitoring indicated that the reaction was completed, and the reaction was quenched by adding water (50 mL). Tetrahydrofuran was recovered under reduced pressure. Then dichloromethane (300 mL) and water (400 mL) were added for extraction. Liquid separation was performed, and the organic phase was dried and precipitated to give a white flocculent solid, which was slurried with *n-*heptane to give pure compound (7-3-a) (77 g) with a yield of 85*%*.

Characterization data for compound (7-3-a): ¹H NMR (400 MHz, Chloroform-*d*) δ 7.60-7.51 (m, 4H), 7.42 (t, J = 7.6 Hz, 2H), 7.32 (dd, J = 14.0, 7.6 Hz, 3H), 5.01 (d, J = 7.7 Hz, 1H), 4.21 (s, 1H), 4.12 (q, J = 7.1 Hz, 2H), 3.65 (dd, J = 10.8, 3.8 Hz, 1H), 3.53 (dt, J = 11.1, 3.6 Hz, 1H), 2.95 (tt, J = 13.4, 6.4 Hz, 2H), 1.24 (t, J = 7.1 Hz, 3H); ¹³C NMR (101 MHz, CDCl₃) δ 155.78, 140.64, 139.76, 135.89, 129.63, 128.73, 127.36, 127.22, 126.93, 61.04, 52.35, 46.75, 28.29, 14.51.

### Step 4: synthesis of compound (8-a)

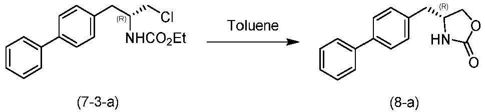

The compound (7-3-a) (70 g) obtained in step 3 was added to a 500 mL reaction flask, and toluene (200 mL) was added. The mixture was heated to 110 °C and stirred at reflux for 2-3 h. (7-3-a) disappeared, and compound (8-a) was generated as monitored by LC-MS. Toluene was recovered under reduced pressure to give a pale yellow solid (63 g), which could be directly used for the subsequent reaction.

### Example 7: Synthesis of Compound (10-a)

This example is a kilogram-level scale-up reaction, and the room temperature of the process is 25-35 °C.

### Step 1: synthesis of compound (2-a)

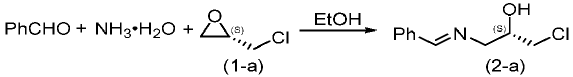

Benzaldehyde (1.0 kg, 9.42 mol) was added to ethanol (4 L), and the mixture was stirred at room temperature. Aqueous ammonia (25*%*, 1 L) was dropwise added to the reaction system at 15-20 °C. After the dropwise addition was completed, the mixture was warmed to room temperature and stirred for 20 min, and then (S)-epichlorohydrin (1.05 kg, 11.3 mol, 1.2 eq) was diluted with ethanol (2 L) and slowly and dropwise added to the reaction system under nitrogen atmosphere. After the dropwise addition was completed, the system was stirred at room temperature (25-30 °C) for 12 h. After epichlorohydrin disappeared as monitored by GC, the reaction was completed. Ethanol was recovered by distillation under reduced pressure to give a yellow oily product, and ethanol (1 L) was added for distillation again to give a pale yellow oily liquid (2.3 kg), which was crude compound (2-a).

### Step 2: synthesis of compound (4-a)

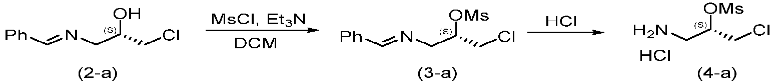

The crude compound (2-a) (2.3 kg, 8.5 mol) obtained in the previous step (step 1 of Example 7) was added to dichloromethane (8 L), and the mixture was stirred at room temperature until a small amount of a white insoluble substance was observed. The mixture was filtered, and triethylamine (1.29 kg, 12.75 mol, 1.5 eq) was added to the filtrate. The mixture was cooled to an internal temperature of 0-10 °C, and methanesulfonyl chloride (1.27 kg, 11.05 mol, 1.3 eq) was dropwise added. The system was heated to an internal temperature of 12-20 °C, and a white solid precipitated. After the dropwise addition was completed, the mixture was warmed to room temperature and stirred for 1 h. Then the starting materials disappeared as monitored by normal phase HPLC. Water (2 L) was added to the system, and the mixture was uniformly stirred for 30 min and extracted. The organic phase was washed twice with water (2 L × 2).

Concentrated hydrochloric acid (1.2 kg) diluted with water (2.4 L) was added to the organic phase treated in the previous step. The mixture was uniformly stirred at room temperature for 3-4 h and left to stand for liquid separation. The organic phase was washed with water (500 mL), and the aqueous phases were combined to give an aqueous solution of crude compound (4-a), which was then used directly for the subsequent reaction.

### Step 3: synthesis of compound (5-a)

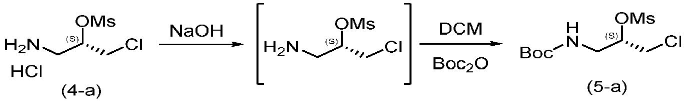

The aqueous solution of the crude compound (4-a) (7.63 mol) obtained in step 2 of Example 7 was added to a reaction kettle and cooled to an internal temperature lower than 10 °C, and then an aqueous sodium hydroxide solution (sodium hydroxide (460 g) dissolved in water (2 L)) was dropwise added. The system gradually turned into a white turbid state, and after the dropwise addition was completed, the system was cooled to 0-10 °C. Di-*tert*-butyl dicarbonate (1.7 kg, 7.63 mol, 1 eq) was dropwise added to the reaction system. The system generated gas and released heat, and the system was heated to an internal temperature of 10-15 °C. After the dropwise addition was completed, the mixture was left to react for 2 h with the temperature maintained. After the starting materials disappeared as monitored by LC-MS, the reaction was completed. The mixture was left to stand for liquid separation, and dichloromethane (2.7 kg) was added to the reaction for extraction. The organic phases were separated after standing, washed with water (2 kg), dried, and filtered, and the solvent was recovered by distillation under reduced pressure to give a pale yellow oily liquid, which became a white waxy solid (2.5 kg) after standing as crude compound (5-a).

### Step 4: synthesis of compound (6-a)

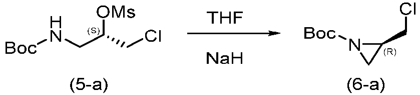

The crude compound (5-a) (2.5 kg, 7.65 mol) obtained in step 3 of Example 7 was added to tetrahydrofuran (5 L) and completely dissolved by stirring at room temperature to give a solution of the compound (5-a) in tetrahydrofuran for later use.

Tetrahydrofuran (2 L) was added to a reaction kettle and cooled to 0-10 °C. A sodium hydride solid (60*%*, 460 g, 11.47 mol, 1.5 eq) was added to the reaction kettle, and the mixture was stirred for 30 min with the temperature maintained. Then the solution of the compound (5-a) in tetrahydrofuran was dropwise added, and the dropwise addition was completed over 2-3 h. The mixture was heated to 50 °C and left to react for 5 h. Then the starting materials disappeared as monitored by LC-MS. The reaction was quenched by adding water (500 mL), and then tetrahydrofuran was recovered under reduced pressure. Dichloromethane (4 L) and water (2.5 L) was added for extraction. The organic phase was dried and precipitated to give a pale yellow oily liquid (1.75 kg). The colorless oily liquid (1.1 kg) was obtained as compound (6-a) by purifying by distillation under reduced pressure with a GC purity of 96*%*, and a total yield of the first five steps was 60*%*.

### Step 5: synthesis of compound (7-a)

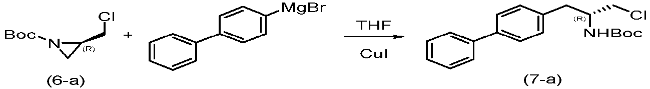

The compound (6-a) (1.1 kg) obtained in step 4 of Example 7 was dissolved in tetrahydrofuran (4.5 L) to give a solution of the compound (6-a) in tetrahydrofuran for later use. Copper(I) iodide (11 g) was added to a dry and oxygen-free reaction kettle, and biphenylmagnesium bromide (3 L, 2.0 M, 6 mol) was added to the reaction system under nitrogen atmosphere. The mixture was cooled to an internal temperature of -15 to -20 °C, and then the solution of the compound (6-a) in tetrahydrofuran was dropwise added to the reaction system. The system gradually changed to grayish green. The dropwise addition was completed over 2 h, and the mixture was left to react for 1-2 h with the temperature maintained. GC monitoring indicated that the reaction was completed, and the reaction was quenched by adding water (2 L). Tetrahydrofuran was recovered under reduced pressure. Then dichloromethane (5 L) and water (6 L) were added for extraction. Liquid separation was performed, and the organic phase was dried and precipitated to give a white flocculent solid (2.1 kg), which was slurried with *n*-heptane to give pure compound (7-a) (1.6 kg) with a yield of 79*%*.

### Step 6: synthesis of compound (10-a)

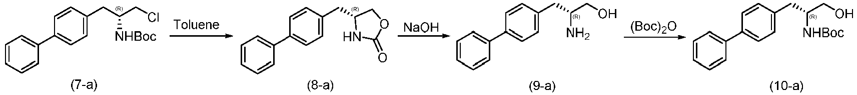

The compound (7-a) (1.6 kg) obtained in step 5 of Example 7 was added to toluene (8 L), and the mixture was heated to 100-110 °C at reflux for 1-2 h. The compound (7-a) disappeared, and compound (8-a) was generated as monitored by LC-MS. The system was cooled and supplemented with toluene (2 L), and then sodium hydroxide (740 g) was added to the reaction. The mixture was left to react at reflux for 3 h. The compound (8-a) disappeared, and compound (9-a) was generated as monitored by LC-MS. The system was cooled to 25-30 °C, and di-*tert*-butyl dicarbonate (1.1 kg) was dropwise added. The system gradually became turbid, and a white solid precipitated. After the dropwise addition was completed, the mixture was left to react for 2 h with the temperature maintained. HPLC indicated the disappearance of compound (9-a) and the generation of compound (10-a). The system was supplemented with water (2 L), heated to 50-60 °C, stirred for 30 min, and left to stand for liquid separation with the temperature maintained. The organic phases were separated and slowly cooled to 0 °C to precipitate a white solid. The solid was filtered and dried to give compound (10-a) (white solid) (1.25 kg) with a purity of 99.3*%*. A total yield for the three steps was 87*%*.

The examples described above are preferred embodiments of the present disclosure, which, however, are not intended to limit the embodiments of the present disclosure. Any other changes, modifications, substitutions, combinations, and simplifications can be made without departing from the spirit and principle of the present disclosure, and should be construed as equivalent replacements and included in the protection scope of the present disclosure.

## Claims

1. A preparation process for compound (10), comprising the following steps: 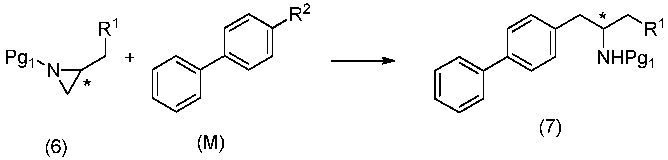
step a: reacting compound (6) with compound (M) under an action of a catalyst to give compound (7), wherein
"*" represents that compound (6) and compound (7) are both in R configuration or S configuration; preferably, "*" represents that compound (6) and compound (7) are both in R configuration;
R¹ is F, Cl, Br, I, or -OR³, and R³ represents C₁₋₆ alkyl or C₁₋₆ heteroalkyl;
R² is 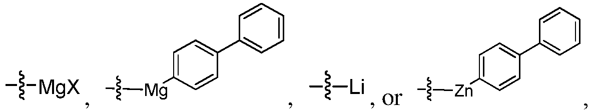 wherein X is Cl, Br, or I;
Pg₁ is an amino protective group; and
compound (10) has a structure: 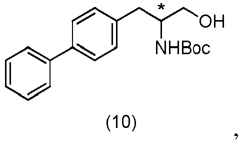 wherein "*" represents that compound (10) is in R configuration or S configuration, preferably, "*" represents that compound (10) is in R configuration.

2. The process according to claim 1, wherein
the catalyst is a cuprous salt, preferably CuI, CuBr, CuCl, or CuCN;
the amino protective group is benzyloxycarbonyl, *tert*-butoxycarbonyl, methoxycarbonyl, ethoxycarbonyl, isopropyloxycarbonyl, isobutyloxycarbonyl, fluorenylmethoxycarbonyl, allyloxycarbonyl, or trimethylsilylethoxycarbonyl; or
the reaction is performed under dry and oxygen-free conditions; preferably, the reaction is performed under dry and oxygen-free conditions with introduction of nitrogen for protection; or
the reaction temperature of the reaction is -60 to 0 °C; preferably, the reaction temperature is -45 to 0 °C; more preferably, the reaction temperature is -30 to -10 °C; most preferably, the reaction temperature is -20 to -15 °C; or
the reaction time of the reaction is 0.5-3 h; preferably, the reaction time is 0.8-2 h; more preferably, the reaction time is 1-1.5 h; most preferably, the reaction time is 1 h.

3. The process according to any one of claims 1-2, wherein the reaction solvent of the reaction is a first solvent, and the first solvent is an organic aprotic solvent; preferably, the organic aprotic solvent is tetrahydrofuran, 2-methyltetrahydrofuran, toluene, cyclopentyl methyl ether, dichloromethane, methyl *tert*-butyl ether, or diethyl ether, or any combination thereof.

4. The process according to any one of claims 1-3, wherein the feeding molar ratio of compound (6) to compound (M) is 1:0.7-2, preferably 1:1-2, more preferably 1:1-1.5, and most preferably 1:1.

5. The process according to any one of claims 1-4, further comprising the following steps: 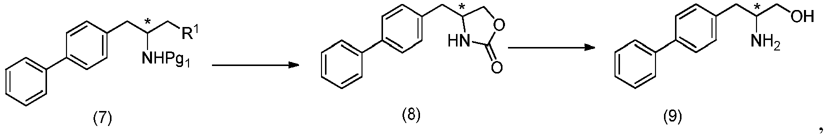
wherein "*" represents that compound (7), compound (8), and compound (9) are all in R configuration or S configuration; preferably, "*" represents that compound (7), compound (8), and compound (9) are all in R configuration;
step b: reacting compound (7) in a second solvent under a first heating condition to give compound (8), wherein
preferably, the reaction time is 0.3-2 h; more preferably, the reaction time is 0.3-1 h; most preferably, the reaction time is 0.5 h;
step c: subjecting compound (8) and a base to a hydrolysis reaction in a third solvent under a second heating condition to give compound (9), wherein
preferably, the base is sodium hydroxide, potassium hydroxide, lithium hydroxide, potassium *tert*-butoxide, sodium *tert*-butoxide, sodium carbonate, potassium carbonate, or sodium methoxide, or any combination thereof;
preferably, the feeding molar ratio of compound (8) to the base is 1:(0.9-5); more preferably, the feeding molar ratio of compound (8) to the base is 1:(1-4); even more preferably, the feeding molar ratio of compound (8) to the base is 1:(2-4); most preferably, the feeding molar ratio of compound (8) to the base is 1:3.75; and
preferably, the reaction time is 1-5 h; more preferably, the reaction time is 2-4 h; most preferably, the reaction time is 3 h.

6. The process according to claim 5, wherein the heating temperatures under the first heating condition and the second heating condition are each independently 80-150 °C, preferably 100-150 °C, further preferably 110-130 °C, and most preferably 120 °C; preferably, the heating temperatures under the first heating condition and the second heating condition are the same temperature; or
the second solvent and the third solvent are each independently *n*-butanol, benzene, toluene, ethylene glycol dimethyl ether, *N*,*N*-dimethylformamide, or *N*,*N*-dimethylacetamide, or any combination thereof; preferably, the second solvent and the third solvent are the same solvent.

7. The process according to claim 5 or 6, wherein the reaction of step c is performed by adding a base directly without any post-treatment after the reaction of step b.

8. The process according to any one of claims 5-7, further comprising the following steps: 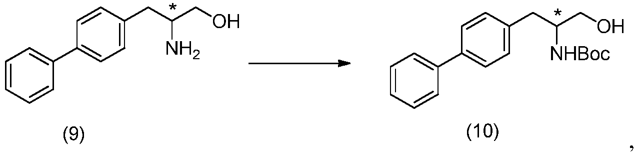
wherein "*" represents that compound (9) and compound (10) are both in R configuration or S configuration; preferably, "*" represents that compound (9) and compound (10) are both in R configuration; and
step d: subjecting compound (9) to an amino protection reaction to give compound (10).

9. The process according to any one of claims 1-8, wherein compound (6) is prepared by the following steps: 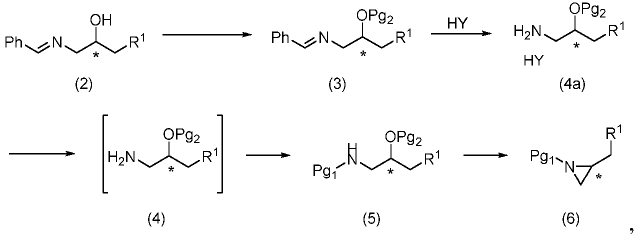
wherein "*" on compound (2), compound (3), compound (4a), compound (4), and compound (5) represents that compound (2), compound (3), compound (4a), compound (4), and compound (5) are all in S configuration, and "*" on compound (6) represents that compound (6) is in R configuration; or "*" on compound (2), compound (3), compound (4a), compound (4), and compound (5) represents that compound (2), compound (3), compound (4a), compound (4), and compound (5) are all in R configuration, and "*" on compound (6) represents that compound (6) is in S configuration;
Pg₂ is a hydroxyl protective group, preferably methanesulfonyl, trifluoromethanesulfonyl, *p*-toluenesulfonyl, or nitrosulfonyl; R¹ and Pg₁ have the definitions according to claim 1;
step a': subjecting compound (2) and a hydroxyl protective reagent to a hydroxyl protection reaction to give compound (3); preferably, the feeding molar ratio of compound (2) to the hydroxyl protective reagent is 1:(1-3); more preferably, the feeding molar ratio of compound (2) to the hydroxyl protective reagent is 1:(1-2); even more preferably, the feeding molar ratio of compound (2) to the hydroxyl protective reagent is 1:(1.2-1.5); most preferably, the feeding molar ratio of compound (2) to the hydroxyl protective reagent is 1:1.3;
step b': reacting compound (3) with acid HY to give compound (4a); preferably, the acid HY is hydrochloric acid, hydrobromic acid, formic acid, hydroiodic acid, *p*-toluenesulfonic acid, or trifluoromethanesulfonic acid;
step c': reacting compound (4a) under an action of a base to give compound (4); preferably, the base is sodium hydroxide, potassium hydroxide, sodium carbonate, or potassium carbonate;
step d': subjecting compound (4) and an amino protective reagent to an amino protection reaction to give compound (5); preferably, the feeding molar ratio of compound (4) to the amino protective reagent is 1:1-1.5; and
step e': reacting compound (5) in a fourth solvent under an action of a basic reagent to give compound (6); preferably, the basic reagent is sodium hydride, sodium methoxide, sodium hydroxide, potassium hydroxide, potassium *tert*-butoxide, sodium *tert*-butoxide, potassium carbonate, sodium carbonate, or sodium ethoxide; preferably, the feeding molar ratio of compound (5) to the basic reagent is 1:(1-3), more preferably 1:(1.5-2.5), and most preferably 1:2.2; preferably, the fourth solvent is tetrahydrofuran or *N*,*N*-dimethylformamide, or a combination thereof; preferably, the reaction temperature is -10 to 50 °C; further preferably, the reaction temperature is -10 to 15 °C; even more preferably, the reaction temperature is -5 to 10 °C; most preferably, the reaction temperature is 0-10 °C.

10. The process according to any one of claims 1-8, wherein compound (6) is prepared by the following steps: 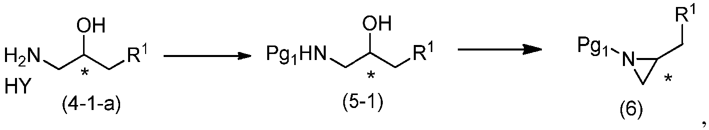 wherein
"*" on compound (4-1-a) and compound (5-1) represents that compound (4-1-a) and compound (5-1) are both in S configuration, and "*" on compound (6) represents that compound (6) is in R configuration; or "*" on compound (4-1-a) and compound (5-1) represents that compound (4-1-a) and compound (5-1) are both in R configuration, and "*" on compound (6) represents that compound (6) is in S configuration;
step a": subjecting compound (4-1-a) and an amino protective reagent to an amino protection reaction under an action of a base to give compound (5-1); preferably, the feeding molar ratio of compound (4-1-a) to the amino protective reagent is 1:(0.7-2); preferably, the base is sodium hydroxide, potassium hydroxide, sodium carbonate, sodium bicarbonate, potassium bicarbonate, or potassium carbonate; and
step b": reacting compound (5-1) at a low temperature under an action of triphenylphosphine and ethyl azodicarboxylate and under nitrogen atmosphere to give compound (6), wherein the low temperature is -40 to 0 °C, preferably -30 to -10 °C, and more preferably -20 to -10 °C.

11. The process according to any one of claims 1-8, wherein compound (6) is prepared by the following steps: 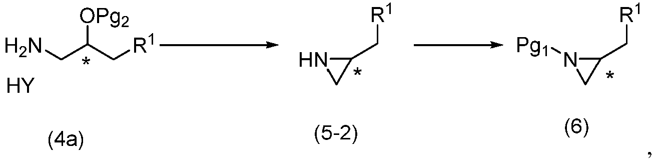 wherein
"*" on compound (4a) represents that compound (4a) is in S configuration, and "*" on compound (5-2) and compound (6) represents that compound (5-2) and compound (6) are both in R configuration; or "*" on compound (4a) represents that compound (4a) is in R configuration, and "*" on compound (5-2) and compound (6) represents that compound (5-2) and compound (6) are both in S configuration;
step a‴: reacting compound (4a) under an action of a base to give compound (5-2); preferably, the base is sodium hydroxide, potassium hydroxide, sodium carbonate, sodium bicarbonate, potassium bicarbonate, or potassium carbonate; preferably, the feeding molar ratio of compound (4a) to the base is 1:(1-3), more preferably 1:(1.2-2.5), and most preferably 1:1.5; preferably, the reaction temperature is 10-100 °C; more preferably, the reaction temperature is 30-80 °C; even more preferably, the reaction temperature is 40-60 °C; most preferably, the reaction temperature is 50 °C; and
step b‴: continuously subjecting compound (5-2) and an amino protective reagent to an amino protection reaction to give compound (6); preferably, the feeding molar ratio of compound (5-2) to the amino protective reagent is 1:(0.7-2); preferably, the reaction is a low-temperature reaction, and the low temperature is -5 to 15 °C; more preferably, the low temperature is 0-10 °C.

12. A compound, having a structure of formula (7a) or (7b) below: 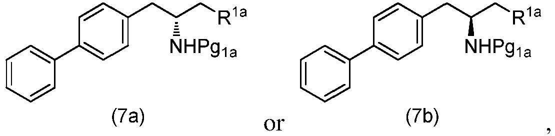
wherein R^{1a} is F, Cl, Br, I, or -OR³, and R³ represents C₁₋₆ alkyl or C₁₋₆ heteroalkyl;
Pg₁ₐ is an amino protective group, preferably benzyloxycarbonyl, *tert*-butoxycarbonyl, methoxycarbonyl, ethoxycarbonyl, isopropyloxycarbonyl, isobutyloxycarbonyl, fluorenylmethoxycarbonyl, allyloxycarbonyl, or trimethylsilylethoxycarbonyl;
and when R^{1a} is Br or I, Pg₁ₐ is not tert-butoxycarbonyl.

13. The compound according to claim 12, having one of the following structures:

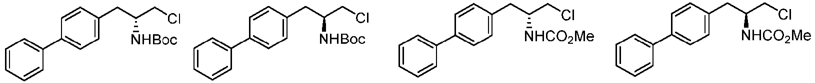

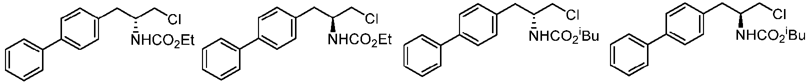

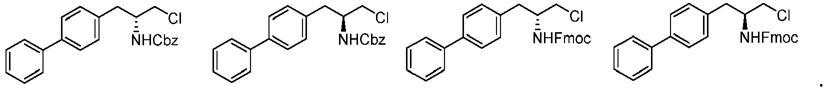

14. A compound, having a structure of formula (6a) or (6b) below: 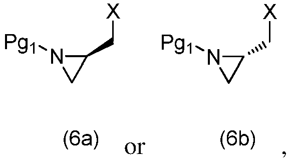
wherein X is F, Cl, Br, or I; and
Pg₁ is an amino protective group, preferably benzyloxycarbonyl, *tert*-butoxycarbonyl, methoxycarbonyl, ethoxycarbonyl, isopropyloxycarbonyl, isobutyloxycarbonyl, fluorenylmethoxycarbonyl, allyloxycarbonyl, or trimethylsilylethoxycarbonyl.

15. The compound according to claim 14, having one of the following structures:

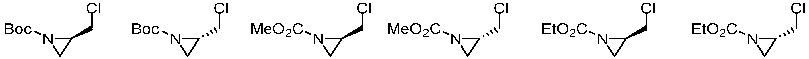

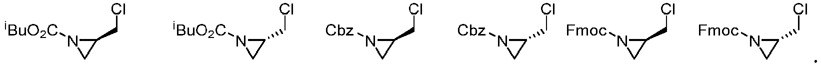

16. A compound, having a structure of formula (8-a) or (8-b) below:

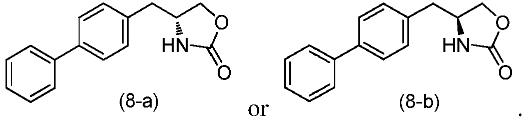

17. Use of the process according to any one of claims 1-11 or the compound according to any one of claims 12-15 in the preparation of sacubitril.
